# EUROPEAN PATENT APPLICATION

(11) **EP 4 365 188 A1**
(43) Date of publication of application: **08.05.2024**
(21) Application number: 22833541.0
(22) Date of filing: 27.06.2022
(51) Int. Cl.: C07K 7/52, C07K 1/107

(54) **METHOD FOR PREPARING MICROCOCCIN-BASED CARBOXYLIC ACID COMPOUND AND MICROCOCCIN-BASED CARBOXYLIC ACID COMPOUND PREPARED THEREBY**

(30) Priority: 30.06.2021 KR 20210085881; 24.06.2022 KR 20220077357
(71) Applicant: A&J Science Co.,Ltd., Daegu 41061 (KR)
(72) Inventor: HWANG, Hee-Jong, Daegu 41106 (KR); SON, Young-Jin, Daegu 41104 (KR); KIM, Dahyun, Daegu 41084 (KR); LEE, Jusuk, Daegu 41119 (KR); CLOVIS, Shyaka, Daegu 41063 (KR); CIUFOLINI, Marco, Vancouver, British Columbia V6K 1V5 (CA)
(74) Representative: Potter Clarkson
(86) International application number: PCT/KR2022/009133
(87) International publication number: WO 2023/277485

(57) **Abstract**

The present invention provides a method for preparing a micrococcin-based carboxylic acid compound and a micrococcin-based carboxylic acid compound prepared thereby, and the method for preparing a micrococcin-based carboxylic acid compound according to the present invention enables easy introduction of various functional groups by introducing a carboxyl group into a micrococcin compound.

## Description

### [Technical Field]

The present invention relates to a method for preparing a micrococcin-based carboxylic acid compound prepared based on a micrococcin compound and a micrococcin-based carboxylic acid compound prepared therefrom.

In addition, the present invention relates to a biosynthesis method of a micrococcin compound which is a starting material for preparing a micrococcin-based carboxylic acid compound.

In addition, the present invention relates to a method for preparing a micrococcin derivative useful as an antibiotic from the micrococcin-based carboxylic acid compound prepared above.

### [Background Art]

Humankind has responded to various infectious diseases by developing antibiotics.

However, recently, a frequency of the appearance of Methicillin resistance Staphylococcus aureus (MRSA) which is resistant to most of the antibiotic substances is increasing.

Methicillin resistance Staphylococcus aureus causes nosocomial infection among patients admitted to hospitals, medical institutions, or the like and spreads from person to person through a body contact, and when it is transmitted through trauma after surgery or causes pneumonia, it is so dangerous that life is at risk.

In addition, tuberculosis which is a chronic infectious disease caused by tuberculosis bacteria including M. *tuberculosis, M. bovis, M. microti,* and *M. africanum* is also one of diseases which has not yet been overcome.

Tuberculosis may remain latently infected for a long time depending on a patient's immune status, and it is estimated that 2 billion people which is about 30% of the world's population are latently infected. When a person is infected with tuberculosis, he/she may be asymptomatic for a considerable period of time but may show acute inflammation of the lungs as a common symptom, and as a result, fever and nonproductive cough are caused. When it is not treated, serious complications and death commonly result.

Development of an antituberculosis drug began with streptomycin in the 1940s and more effective antituberculosis drugs were developed, and as a result, the incidence of tuberculosis and deaths resulting therefrom were rapidly decreased.

However, due to the incidence of "multidrug-resistant tuberculosis (MDR-TB)" which is a resistant strain which may not be controlled with existing antibiotics in the 1980s, the number of patients with multidrug-resistant tuberculosis has not decreased, which emerged as a serious health problem.

Meanwhile, micrococcin is a thiopeptide natural product, has a structural feature of having a macrocycle composed of 26 atoms, and is classified into micrococcin Pa (MP1) and micrococcin P2 (MP2). Micrococcin P1 is known to have efficacy against various gram-positive bacteria, and shows an antibacterial action with a mechanism of action to bind to a composite formed between L11 protein and 23S rRNA and inhibit protein synthesis.

Thus, a study for developing a micrococcin derivative having an improved antibiotic effect is in progress.

However, since micrococcin has a complicated structure which contains a plurality of thiazole rings and is produced by microorganisms, it is not easy to introduce various functional groups.

### [Disclosure]

### [Technical Problem]

Thus, the inventors of the present invention studied in order to introduce various functional groups to micrococcin, and found that a micrococcin-based carboxylic acid compound may be prepared by reacting a specific micrococcin compound with a metal hydroxide to cut out a specific functional group, thereby completing the present invention.

An object of the present invention is to provide a method for preparing a micrococcin-based carboxylic acid compound.

Another object of the present invention is to provide a biosynthesis method of a micrococcin compound which is a starting material for preparing a micrococcin-based carboxylic acid compound.

Still another object of the present invention is to provide a method for preparing a micrococcin derivative useful as an antibiotic from the micrococcin-based carboxylic acid compound prepared above.

### [Technical Solution]

In one general aspect, a method for preparing a micrococcin-based carboxylic acid compound which is useful as an intermediate for preparing a micrococcin derivative having an excellent antibiotic effect is provided.

The method for preparing a micrococcin-based carboxylic acid compound of the present invention includes:

reacting a micrococcin compound represented by the following Chemical Formula 2 with a metal hydroxide to prepare a micrococcin-based carboxylic acid compound represented by the following Chemical Formula 1: 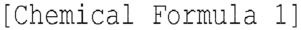 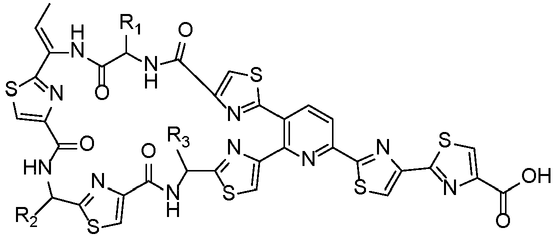 [23] [24] 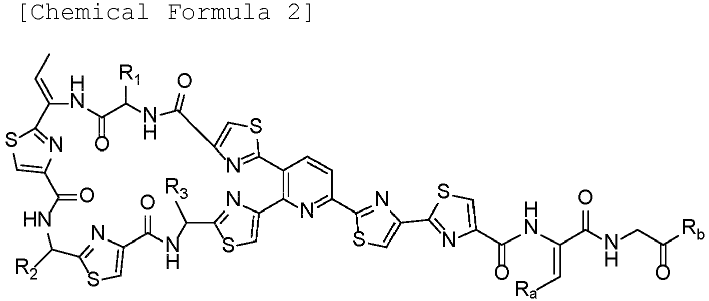 wherein
R₁ to R₃ are independently of one another hydrogen, C1-C10 alkyl, C3-C10 cycloalkyl, C6-C12 aryl, or C3-C10 heteroaryl, and
the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl;
Rₐ is hydrogen, C1-C10 alkyl, C2-C10 alkenyl, or C1-C10 alkoxyC1-C10 alkyl; and
R_{b} is hydrogen or C1-C10 alkyl.

In another general aspect, a biosynthesis method of a micrococcin compound which is a starting material for preparing a micrococcin-based carboxylic acid compound is provided.

The biosynthesis method of a micrococcin compound of the present invention, which is a method for preparing a micrococcin compound of the following Chemical Formula 2-2, includes:
1) producing an expression vector including BcTclE, BcTclI, BcTclJ, BcTclK, BcTclL, BcTclM, BcTclN, and BcTclP genes; and
2) transforming Bacillus subtilis with the expression vector of 1):
   [35] 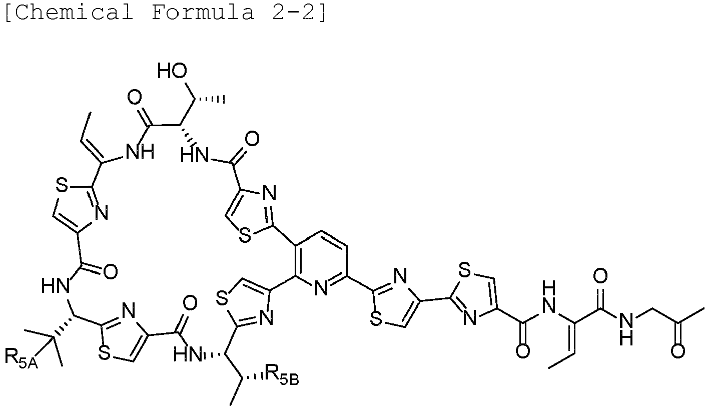 wherein
   R_{5A} is hydrogen or hydroxy, and R_{5B} is hydroxy or methoxy.

In another general aspect, a micrococcin-based carboxylic acid compound which is useful as an intermediate for preparing a micrococcin derivative which may have an excellent antibiotic effect represented by Chemical Formula 1 is provided.

In still another general aspect, a method for preparing a micrococcin derivative useful as an antibiotic using the micrococcin-based carboxylic acid compound prepared above as an intermediate is provided.

The method for preparing a micrococcin derivative of the present invention includes:
reacting a micrococcin compound of Chemical Formula 2 with a metal hydroxide to prepare a micrococcin-based carboxylic acid compound of Chemical Formula 1; and
preparing a micrococcin derivative of the following Chemical Formula 3 from the micrococcin-based carboxylic acid compound of Chemical Formula 1:
   [44] 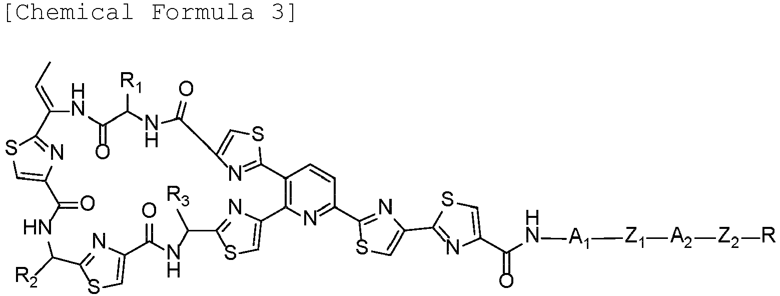 wherein
   R₁ to R₃ are independently of one another hydrogen, C1-C10 alkyl, C3-C10 cycloalkyl, C6-C12 aryl, or C3-C10 heteroaryl;
   the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl;
   Z₁ and Z₂ are independently of each other a single bond, -CONR₁₁-, -NR₁₂CO-, -COO-, -OCO-, -CR₁₃R₁₄-, -NR₁₅COO-, -NR₁₆-, -S-, -O-, -SO₂-, or -OCONR₁₇-, in which R₁₁ to R₁₇ are independently of one another hydrogen, hydroxy, C1-C10 alkyl, carboxylC1-C10 alkyl, or C1-C10 alkoxycarbonylC1-C10 alkyl;
   A₁ is 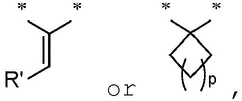 in which R' is hydrogen, C1-C10 alkyl, C2-C10 alkenyl, or C1-C10 alkoxyC1-C10 alkyl, and p is an integer of 0 to 4;
   A₂ is a single bond, C1-C10 alkylene, C3-C10 cycloalkylene, C3-C10 heterocycloalkylene, C6-C20 arylene, or C6-C20 heteroarylene; and
   R is hydrogen, halogen, amino, hydroxy, -B(OH)₂, substituted or unsubstituted haloC1-C10 alkyl, substituted or unsubstituted C1-C10 alkyl, substituted or unsubstituted C2-C10 alkenyl, substituted or unsubstituted C3-C10 cycloalkyl, substituted or unsubstituted C3-C10 heterocycloalkyl, substituted or unsubstituted C6-C20 aryl, or substituted or unsubstituted C3-C20 heteroaryl.

### [Advantageous Effects]

The method for preparing a micrococcin-based carboxylic acid compound of the present invention allows easy preparation of a compound to which a micrococcin-based carboxylic acid compound is introduced from a micrococcin compound by a simple process under mild conditions.

The method for preparing a micrococcin-based carboxylic acid compound of the present invention allows preparation of a micrococcin-based carboxylic acid compound in high purity and yield under mild conditions.

In addition, the micrococcin-based carboxylic acid compound prepared by the method for preparing a micrococcin-based carboxylic acid compound of the present invention allows easy preparation of various micrococcin derivatives by easily introducing various functional groups to an introduced carboxylic acid group. That is, the micrococcin-based carboxylic acid compound of the present invention may be very useful as an intermediate for preparing a micrococcin derivative to which various functional groups are introduced.

Therefore, the method for preparing a micrococcin-based carboxylic acid compound of the present invention may be very useful for preparing a micrococcin-based derivative having an excellent antibiotic effect.

In addition, according to the present invention, a micrococcin compound which is a target material may be efficiently biosynthesized in Bacillus subtilis cells transformed with a vector including specific genes, and various substituents may be introduced to a macrocycle by further introducing a useful gene.

### [Description of Drawings]

FIG. 1 - a schematic diagram of a genetic recombination plasmid 1 introduced to pUC19 of Example 2 of the present invention.
FIG. 2 - a schematic diagram of a genetic recombination plasmid 2 introduced to pUC19 of Example 2 of the present invention.
FIG. 3 - a schematic diagram of a genetic recombination plasmid 3 introduced to pUC19 of Example 2 of the present invention.
FIG. 4 - UPLC-MS analysis results of a Bacillus extract transformed by each plasmid.

### [Best Mode]

Hereinafter, a method for preparing a micrococcin-based carboxylic acid compound of the present invention will be described in detail. Here, technical terms and scientific terms used in the present specification have the general meaning understood by those skilled in the art to which the present invention pertains unless otherwise defined, and description for the known function and configuration which may unnecessarily obscure the gist of the present invention will be omitted in the following description.

The following terms used in the present specification are defined as follows, but they are only illustrative and do not limit the present invention, application, or use.

The "micrococcin compound of the present invention" which is the term used in the present specification is any compound as long as it is a micrococcin compound recognized by a person skilled in the art, and as an example, has a meaning including not only each compound of Chemical Formulae 11 and 12 which is a micrococcin P2 compound but also clathrates, hydrates, solvates, prodrugs, or polymorphs thereof:

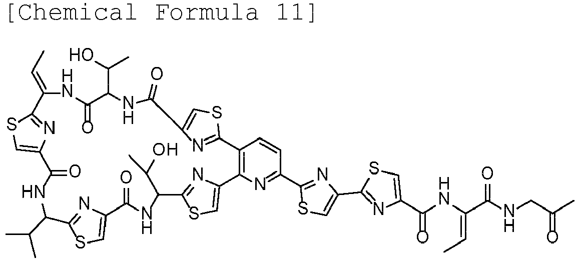

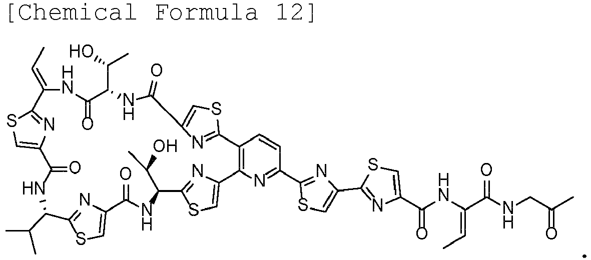

In addition, the terms "micrococcin compound", "micrococcin-based carboxylic acid compound", and "micrococcin derivative" in the present specification have a meaning also including a pharmaceutically acceptable salt of the compound of the present invention, if the pharmaceutically acceptable salt thereof is not mentioned. In an exemplary embodiment, the micrococcin compound of the present invention may exist as a stereoisomerically pure compound (for example, substantially having no other stereoisomer (e.g., 85% ee or more, 90% ee or more, 95% ee or more, 97% ee or more, or 99% ee or more)). That is, when the "micrococcin compound", "micrococcin-based carboxylic acid compound", "micrococcin derivative", or a salt thereof according to the present invention is tautomers and/or stereoisomers (for example, geometrical isomers or conformational isomers), each of their separated isomers and mixtures is also included in the scope of the compound of the present invention. When the compound of the present invention or the salt thereof has asymmetric carbon in the structure, an optical active compound and a racemic compound thereof are also included in the scope of the compound of the present invention. For example, when the compounds of the present invention have a sulfoxide (SOR) structure, they may have chirality. The compound of the present invention includes R and S forms of the isomers, and mixtures of the R and S forms are also included in the scope of the compound of the present invention. In addition, the compounds of the present invention may exist in any one form of a keto form or an enol form, and the forms are also included in the scope of the compound of the present invention.

In the present specification, the terms "substituent", "radical", "group", "moiety", and "fragment" may be used interchangeably.

In the present specification, "C_{A}-C_{B}" refers to "the number of carbons being A or more and B or less". That is, when "C1-C10" is described, it means that the number of carbon atoms is 1 to 10. For example, C1-C10 alkyl refers to alkyl having 1 to 10 carbon atoms.

The term "alkyl" used in the present specification refers to a saturated straight chain or branched chain acyclic hydrocarbon having 1 to 20, preferably 1 to 15, more preferably 1 to 10, more preferably 1 to 6 carbon atoms (where the number of carbon atoms is not particularly limited) . "Lower alkyl" refers to straight chain or branched chain alkyl having 1 to 4 or 1 to 6 carbon atoms. Representative saturated straight chain alkyl includes methyl, ethyl, n-propyl, n-butyl, n-pentyl, n-hexyl, n-heptyl, n-octyl, n-nonyl, and n-decyl, while saturated branched chain alkyl includes isopropyl, sec-butyl, isobutyl, tert-butyl, isopentyl, 2-methylhexyl, 3-methylbutyl, 2-methylpentyl, 3-methylpentyl, 4-methylpentyl, 2-methylhexyl, 3-methylhexyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimethylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, and 3,3-diethylhexyl.

The term "alkenyl" used in the present specification refers to a saturated straight chain or branched chain acyclic hydrocarbon including 2 to 20, preferably 2 to 15, preferably 2 to 10, and more preferably 2 to 6 carbon atoms and at least one carbon-carbon double bond. Representative straight chain or branched chain (C2-C10)alkenyl includes vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl, and 3-decenyl. The alkenyl group may be optionally substituted.

The terms "halogen" and "halo" used in the present specification refer to fluorine, chlorine, bromine, or iodine.

The terms "haloalkyl", "haloalkoxy", or "haloalkenyl" used in the present specification refer to an alkyl, alkoxy, or alkenyl group in which one or more hydrogen atoms are substituted by a halogen atom, respectively. For example, the haloalkyl includes -CF₃, -CHF₂, -CH₂F, -CBr₃, -CHBr₂, - CH₂Br, -CCl₃, -CHCl₂, -CH₂CI, -CI₃, -CHI₂, -CH₂I, -CH₂-CF₃, - CH₂-CHF₂, -CH₂-CH₂F, -CH₂-CBr₃, -CH₂-CHBr₂, -CH₂-CH₂Br, -CH₂-CCl₃, -CH₂-CHCl₂, -CH₂-CH₂CI, -CH₂-CI₃, -CH₂-CHI₂, -CH₂-CH₂I, and the like. Herein, alkyl and halogen are as defined above.

The term "alkoxy" used in the present specification refers to -O- (alkyl) including -OCH₃, -OCH₂CH₃, -O(CH₂)₂CH₃, -O(CH₂)₃CH₃, -O(CH₂)₄CH₃, -O(CH₂)₅CH₃, and the like, in which alkyl is as defined above.

The term "lower alkoxy" used in the present specification refers to -O-(lower alkyl), in which the lower alkyl is as defined above.

The term "aryl" used in the present specification refers to a carbocyclic aromatic group containing 5 to 10 ring atoms. A representative example includes phenyl, tolyl, xylyl, naphthyl, tetrahydronaphthyl, anthracenyl, fluorenyl, indenyl, azulenyl, and the like, but is not limited thereto. The carbocyclic aromatic group may be optionally substituted.

The term "cycloalkyl" used in the present specification refers to a monocyclic or polycyclic saturated ring having carbon and hydrogen atoms and no carbon-carbon multiple bond. An example of the cycloalkyl group includes (C3-C10) cycloalkyl, for example, cyclopropyl, cyclobutyl, cyclopentyl, cyclohexyl, and cycloheptyl, but is not limited thereto. The cycloalkyl group may be optionally substituted. In an exemplary embodiment, the cycloalkyl group is a monocyclic or bicyclic ring.

The term "heterocyclyl" used in the present specification refers to a stable 3- to 18-membered saturated or partially unsaturated radical consisting of 2 to 20, preferably 2 to 15, preferably 2 to 10, preferably 2 to 6 carbon atoms and 1 to 6 heteroatoms, for example, 1 to 5 heteroatoms, 1 to 4 heteroatoms, 1 to 3 heteroatoms, or 1 or 2 heteroatoms selected from the group consisting of nitrogen, oxygen, and sulfur. An exemplary heterocycloalkyl includes an unlimitedly stable 3- to 15-membered saturated or partially unsaturated radical, a stable 3- to 12-membered saturated or partially unsaturated radical, a stable 3- to 9-membered saturated or partially unsaturated radical, a stable 8-membered saturated or partially unsaturated radical, a stable 7-membered saturated or partially unsaturated radical, a stable 6-membered saturated or partially unsaturated radical, or a stable 5-membered saturated or partially unsaturated radical. Heterocycloalkyl includes all of saturated or unsaturated monocycle, polycycle, and spyrocycle forms, and may be bonded through a heteroatom or a carbon atom. An example of the heterocycloalkyl radical may include monovalent radicals of non-aromatic heterocycles such as oxetane, aziridine, pyrrolidine, azetidine, piperidine, tetrahydropyridine, piperazine, morpholine, thiomorpholine, 1,3-dihydrobenzo[c] [1,2]oxaborole, imidazolidine-2,4-dione, thiazolidine-2,4-dione, pyrimidine-2,4(1H,3H)-dione, 3-azabicyclo[3.1.0]hexane, octahydropyrrolo[3,4-c]pyrrole, 2,7-diazaspiro[4.4]nonane, and 2-azaspiro[4.4]nonane.

The term "mono-alkylamino" used in the present specification refers to -NH(alkyl) including -NHCH₃, - NHCH₂CH₃, -NH(CH₂)₂CH₃, -NH(CH₂)₃CH₃, -NH(CH₂)₄CH₃, -NH(CH₂)₅CH₃, and the like, wherein alkyl is as defined above.

The term "di-alkylamino" used in the present specification refers to -N(alkyl)(alkyl) including -N(CH₃)₂, -N(CH₂CH₃)₂, -N((CH₂)₂CH₃)₂, -N(CH₃)(CH₂CH₃), and the like, wherein each alkyl is independently alkyl defined above.

The term "alkylamino" used in the present specification has a concept including mono-alkylamino, di-alkylamino, and tri-alkylamino defined above.

The terms "carboxylic acid", "carboxyl", and "carboxy" used in the present specification refers to -COOH.

The term "carboxylalkyl" used in the present specification refers to alkyl in which one or more hydrogen atoms are substituted by hydroxy, including -CH₂COOH, - CH₂CH₂COOH, - (CH₂)₂CH₂COOH, - (CH₂)₃CH₂COOH, - (CH₂)₄CH₂COOH, - (CH₂)₅CH₂COOH, -CH(COOH)-CH₃, -CH₂CH(COOH)CH₃, and the like, wherein alkyl is as defined above.

The term "aminoalkyl" used in the present specification refers to - (alkyl)-NH₂ including -CH₂-NH₂, -(CH₂)₂-NH₂, - (CH₂)₃-NH₂, -(CH₂)₄-NH₂, -(CH₂)₅-NH₂, and the like, wherein alkyl is as defined above.

The term "mono-alkylaminoalkyl" used in the present specification refers to -(alkyl)-NH(alkyl), including -CH₂-NH-CH₃, -CH₂-NHCH₂CH₃, -CH₂-NH(CH₂)₂CH₃, -CH₂-NH(CH₂)₃CH₃, -CH₂-NH(CH₂)₄CH₃, -CH₂-NH(CH₂)₅CH₃, -(CH₂)₂-NH-CH₃, and the like, wherein each alkyl is independently alkyl defined above.

The term "heterocycle (hetero ring)" used in the present specification refers to a saturated or unsaturated 5- to 7-membered monocyclic or 7- to 10-membered bicyclic, heterocyclic ring containing 1 to 4 heteroatoms independently selected from nitrogen, oxygen, and sulfur, in which nitrogen and sulfur heteroatoms may be optionally oxidized, a nitrogen heteroatom may be optionally quaternized, and a bicyclic ring in which some of the heterorings are fused to a benzene ring is included. The heterocycle may be attached by a heteroatom or a carbon atom. The heterocycle includes heteroaryl defined above. Representative heterocycle includes morpholinyl, pyrrolidinonyl, pyrrolidinyl, piperidinyl, hydantoinyl, valerolactamyl, oxiranyl, oxetanyl, tetrahydrofuranyl, tetrahydropyranyl, tetrahydropyridinyl, tetrahydropyrimidinyl, tetrahydrothiophenyl, tetrahydrothiopyranyl, tetrahydropyrimidinyl, tetrahydrothiophenyl, and tetrahydrothiopyranyl. "Heterocycle fused to phenyl" refers to a heterocycle attached to two adjacent carbon atoms of a phenyl ring, in which the heterocycle is as defined above.

"Heteroaryl" used in the present specification is a 5-to 10-membered aromatic heterocycle containing at least one carbon atoms including a mono- and bicyclic ring system and having at least one heteroatom selected from the group consisting of nitrogen, oxygen, and sulfur. Representative heteroaryl is triazolyl, tetrazolyl, oxadiazolyl, pyridyl, furyl, benzofuranyl, thiophenyl, benzothiophenyl, quinolinyl, pyrrolyl, indolyl, oxazolyl, benzoxazolyl, imidazolyl, benzimidazolyl, thiazolyl, benzothiazolyl, isoxazolyl, pyrazolyl, isothiazolyl, pyridazinyl, pyrimidinyl, pyrizinyl, triazinyl, cinnolinyl, phthalazinyl, quinazolyidyl, pyrimidyl, oxetanyl, azepinyl, piperazinyl, morpholinyl, dioxanyl, thietanyl, and oxazolyl. A heteroaryl group may be monocyclic or bicyclic. The heteroaryl may be used interchangeably with a heteroaryl ring, a heteroaryl group, or heteroaromatic, and these terms may all include an optionally substituted ring.

The term "hydroxyalkyl" used in the present specification refers to alkyl in which one or more hydrogen atoms are substituted by hydroxy, including -CH₂OH, -CH₂CH₂OH, - (CH₂)₂CH₂OH, -(CH₂)₃CH₂OH, -(CH₂)₄CH₂OH, -(CH₂)₅CH₂OH, - CH(OH)-CH₃, -CH₂CH(OH)CH₃, and the like, wherein alkyl is as defined above.

The term "hydroxyaryl" used in the present specification refers to aryl in which one or more hydrogen atoms are substituted by hydroxy, wherein aryl is as defined above.

The term "alkylthio" used in the present specification includes -S-CH₃, -S-CH₂CH₃, -S-(CH₂)₂CH₃, -S-(CH₂)₃CH₃, -S-(CH₂)₄CH₃, -S-(CH₂)₃CH₃, and the like, wherein alkyl is as defined above.

The term "sulfonic acid" and "sulfo" used in the present specification refers to -SO₃H,

The term "sulfonylalkyl" used in the present specification refers to -SO₂-(alkyl) including SO₂-CH₃, -SO₂-CH₂CH₃, -SO₂- (CH₂)₂CH₃, -SO₂- (CH₂)₃CH₃, -SO₂- (CH₂)₄CH₃, and -S0₂-(CH₂)₅CH₃, wherein alkyl is as defined above.

The term "sulfinylalkyl" used in the present specification refers to -SO-(alkyl) including -SO-CH₃, -SOCH₂CH₃, -SO- (CH₂)₂CH₃, -SO-(CH₂)₃CH₃, -SO-(CH₂)₄CH₃, -SO-(CH₂)₅CH₃, and the like, wherein alkyl is as defined above.

The term "thioalkyl" used in the present specification includes -S-CH₃, -S-CH₂CH₃, -S-(CH₂)₂CH₃, -S-(CH₂)₃CH₃, -S-(CH₂)₄CH₃, -S-(CH₂)₃CH₃, and the like, wherein alkyl is as defined above.

The term "substitution" of the present specification means that a hydrogen atom bonded to a carbon atom of a compound is changed into another substituent, and a substituted position is not limited as long as it is a position at which a hydrogen atom is substituted, that is, a position at which a substituent may be substituted, and when two or more are substituted, two or more substituents may be the same as or different from each other.

The term "substituted" used in the present specification means that a hydrogen atom of a substituted part (for example, alkyl, aryl, heteroaryl, heterocycle, or cycloalkyl) is replaced with other atoms or other functional groups (that is, substituent). In the case of a keto substituent, two hydrogen atoms are substituted by oxygen which is attached to carbon by a double bond. Unless otherwise stated regarding the substituent, the optionally substituted substituent of the present invention may be halogen, hydroxyl, (lower)alkyl, haloalkyl, mono- or di-alkylamino, aryl, heterocycle, -NO₂, -NRₐ₁R_{b1}, -NRₐ₁C(=O)R_{b1}, -NRₐ₁C(=O)NRₐ₁R_{b1}, -NRₐ₁C (=O)OR_{b1}, -NRₐ₁SO₂R_{b1}, -ORₐ₁, -CN, - C(=O)Rₐ₁, -C(=O)ORₐ₁, -C(=O)NRₐ₁R_{b1}, -OC(=O)Rₐ₁, -OC(=O)ORₐ₁, - OC(=O)NRₐ₁R_{b1}, -NRₐ₁SO₂R_{b1}, -PO₃Rₐ₁, -PO(ORₐ₁)(OR_{b1}), -SO₂Rₐ₁, - S(O)Rₐ₁, -SO(=NRₐ₁)R_{b1} (for example, sulfoximine), -S(=NRₐ₁)R_{b1} (for example, sulfilimine), and -Sra1, ₐ₁, wherein Rₐ₁ and R_{b1} are the same as or different from each other, and independently of each other hydrogen, halogen, amino, alkyl, alkoxyalkyl, haloalkyl, aryl, or heterocycle, or Rₐ₁ and R_{b1} may be a heterocycle form like an attached nitrogen atom. Herein, the number of Rₐ₁ and R_{b1} may be plural depending on the bonded atom, and alkyl may be C1-C20 alkyl, aryl may be C6-C20, and heterocycle may be C3-C20.

The present invention provides a method for preparing a micrococcin-based carboxylic acid compound which may produce a micrococcin derivative having a surprising antibiotic effect, and
the method for preparing a micrococcin-based carboxylic acid compound of the present invention includes:
reacting a micrococcin compound represented by the following Chemical Formula 2 with a metal hydroxide to prepare a micrococcin-based carboxylic acid compound represented by the following Chemical Formula 1:
   [102] 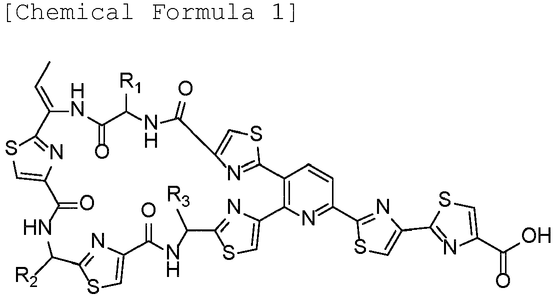 [103] 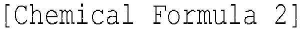 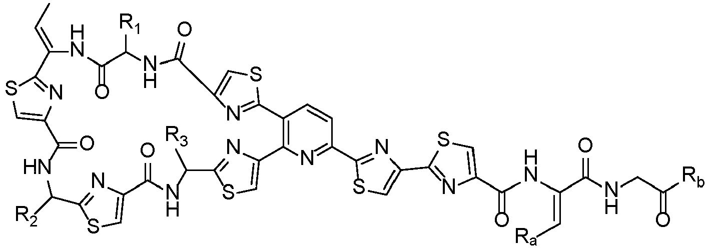 wherein
   R₁ to R₃ are independently of one another hydrogen, C1-C10 alkyl, C3-C10 cycloalkyl, C6-C12 aryl, or C3-C10 heteroaryl, and
   the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl;
   Rₐ is hydrogen, C1-C10 alkyl, C2-C10 alkenyl, or C1-C10 alkoxyC1-C10 alkyl; and
   R_{b} is hydrogen or C1-C10 alkyl.

In the method for preparing a micrococcin-based carboxylic acid compound of the present invention, a micrococcin-based carboxylic acid compound prepared by reacting a specific micrococcin compound with a metal hydroxide to cut out a specific functional group 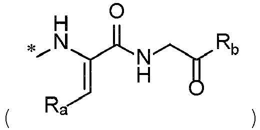 to introduce a hydroxyl group allows various functional groups having excellent activity to be easily introduced.

Specifically, the micrococcin compound of Chemical Formula 2 includes a carbonyl group at the end, the carbonyl group present at the end may form an intramolecular hydrogen bond with oxygen in an amide functional group (-C(=O)-NH-) bonded to thiazole by treating the metal hydroxide, and the formed hydrogen bond may activate carbonyl bonded to a thiazole ring to derive nucleophilic addition of a hydroxide ion. Thus, a nitrogen atom in the amide functional group (-C(=O)-NH-) bonded to thiazole has a significantly lowered basicity and may act as a significantly good leaving group.

In an exemplary embodiment of the present invention, R₁ to R₃ may be independently of one another hydrogen or C1-C10 alkyl, and the alkyl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl,

More preferably, R₁ to R₃ may be independently of one another hydrogen or C1-C5 alkyl, and the alkyl of R₁ to R₃ may be further substituted by one or more selected from the group consisting of hydroxy, thio, C1-C5 alkylthio, C1-C5 alkyl, C1-C5 alkoxy, hydroxyC1-C5 alkyl, C6-C12 aryl, hydroxyC6-C12 aryl, and C3-C8 heteroaryl.

Preferably, in an exemplary embodiment of the present invention, Rₐ may be hydrogen or C1-C10 alkyl; R_{b} may be C1-C10 alkyl.

Preferably, in an exemplary embodiment of the present invention, R₁ to R₃ may be independently of one another hydrogen or *-L₁-R₄, in which L₁ is C1-C3 alkylene, and R₄ is hydrogen, hydroxy, thio, C1-C4 alkylthio, C1-C4 alkyl, C1-C4 alkoxy, C6-C12 aryl, hydroxyC6-C12 aryl, or C3-C10 heteroaryl.

More preferably, in an exemplary embodiment of the present invention, R₁ to R₃ may be independently of one another hydrogen, or any one selected from the following structures:

*-CH₃ *-CH₂OH *-CHCH₃OH *-CHCH₃OCH₃ *-CH₂SH *-C(CH₃)₂OH

*-C(CH₃)₃ *-CH(CH₃)₂ *-CH₂CH(CH₃)₂ *-CHCH₃CH₂CH₃ *-CH₂CH₂SCH₃

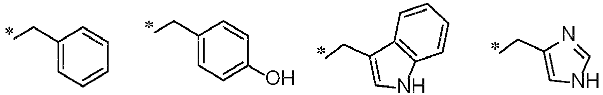

More preferably, Chemical Formula 2 according to an exemplary embodiment of the present invention may be represented by the following Chemical Formula 2-1: 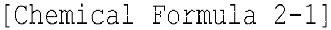 [120] 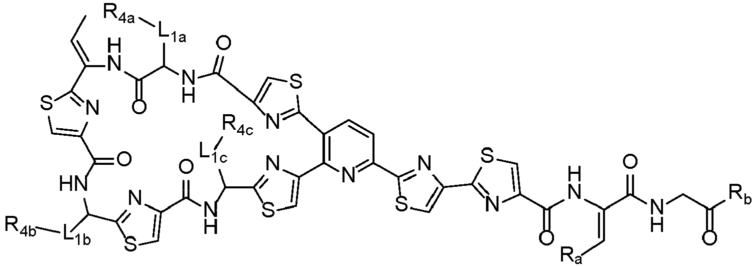 wherein
Rₐ is hydrogen or C1-C10 alkyl;
R_{b} is C1-C10 alkyl;
L₁ₐ to L_{1c} are independently of one another C1-C3 alkylene; and
R₄ₐ to R_{4c} are independently of one another hydrogen, hydroxy, thio, C1-C10 alkylthio, C1-C10 alkyl, C1-C10 alkoxy, hydroxyC1-C10 alkyl, C6-C12 aryl, hydroxyC6-C12 aryl, or C3-C10 heteroaryl.

Preferably, in Chemical Formula 2A, Rₐ may be C1-C5 alkyl, and R_{b} may be C1-C5 alkyl; L₁ₐ to L_{1c} may be independently of one another C1-C3 alkylene; and R₄ₐ to R_{4c} may be independently of one another hydrogen, hydroxy, thio, C1-C5 alkylthio, C1-C5 alkyl, C1-C5 alkoxy, hydroxyC1-C5 alkyl, phenyl, naphthyl, hydroxyphenyl, imidazolyl, or indolyl, and more preferably, in Chemical formula 2A, Rₐ may be C1-C3 alkyl, R_{b} may be C1-C3 alkyl; L₁ₐ to L_{1c} may be independently of one another methylene, 1,1-ethylene, 1,2-ethylene, 1,1-propylene, 1,2-propylene, 1,3-propylene, or 2,2-propylene; and R₄ₐ to R_{4c} may be independently of one another hydrogen, hydroxy, thio, C1-C3 alkylthio, C1-C3 alkyl, hydroxyC1-C3 alkyl, phenyl, naphthyl, hydroxyphenyl, imidazolyl, or indolyl.

In a specific example, *-L₁ₐ-R₄ₐ, *-L_{1b}-R_{4b}, and *-L_{1c}-R_{4c} may be independently of one another any one selected from the following structures:

*-CH₃ *-CH₂OH *-CHCH₃OH *-CHCH₃OCH₃ *-CH₂SH *-C(CH₃)₂OH

*-C(CH₃)₃ *-CH(CH₃)₂ *-CH₂CH(CH₃)₂ *-CHCH₃CH₂CH₃ *-CH₂CH₂SCH₃

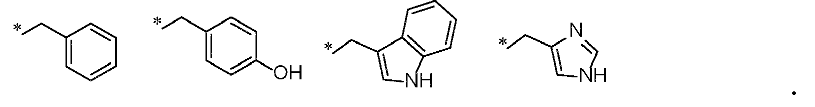

The micrococcin compound according to an exemplary embodiment of the present invention may be selected from the following Chemical Formulae 2-1-A, 2-1-B, and 2-1-C, but is not limited thereto:

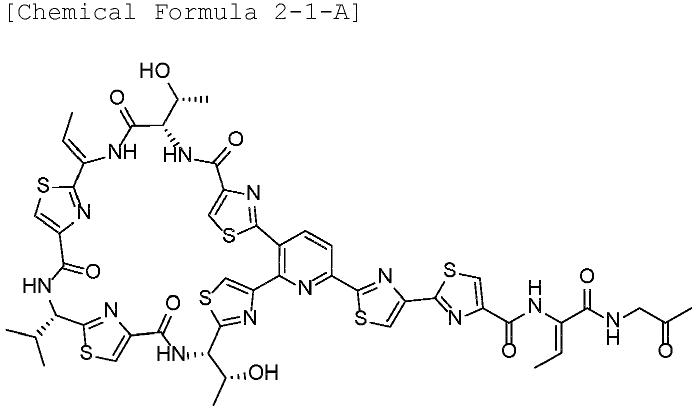

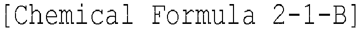

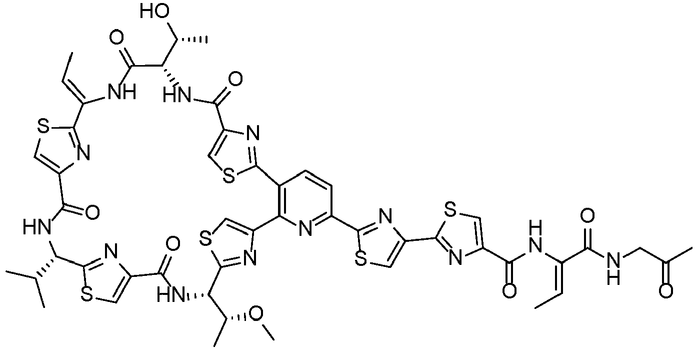

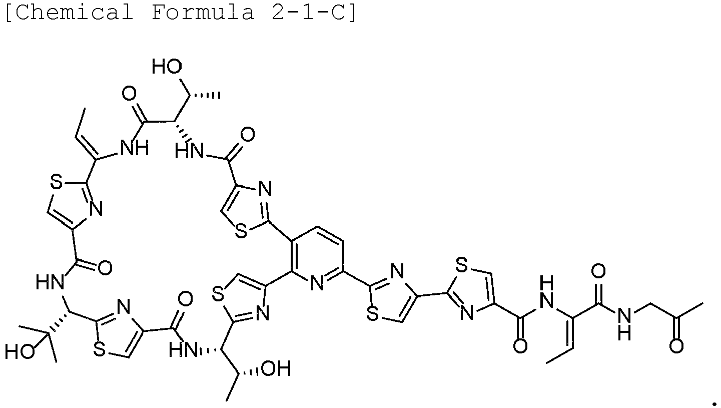

Since the method for preparing a micrococcin-based carboxylic acid compound may easily introduce various functional groups to a micrococcin-based compound by introducing a carboxyl group to a micrococcin skeleton by a simple process under mild conditions, it is very useful for synthesizing various micrococcin derivatives showing an excellent antibiotic effect.

Preferably, the metal of the metal hydroxide according to an exemplary embodiment may be an alkaline metal or an alkali earth metal, but is not limited thereto, and more preferably, may be an alkaline metal, and specifically, may be Li or Na.

The metal hydroxide according to an exemplary embodiment may be used at 3 to 12 mol, preferably 5 to 10 mol with respect to 1 mol of the micrococcin compound, and the reaction may be performed at 30 to 80°C for 8 to 24 hours, preferably 40 to 60°C for 12 to 24 hours. When the metal hydroxide is used in the range described above, a carboxylic acid compound to be desired may be prepared in high purity and yield.

The micrococcin compound of Chemical Formula 2 according to an exemplary embodiment may be separated from a natural product or prepared entirely synthetically, and thus, is not limited thereto.

In a specific example, the micrococcin compound of Chemical Formula 2 may be synthesized by a method available in an organic synthesis field. When the micrococcin compound of Chemical formula 2 of the present invention is a synthesized one, the micrococcin compound of Chemical Formula 2 may be prepared by any method recognized by a person skilled in the art.

As an example, it may be prepared by an aryl-aryl coupling reaction such as Moto coupling, Suzuki coupling, Stille coupling, Sonogashira coupling, Heck coupling, or Buchwald coupling.

Preferably, the aryl-aryl coupling may be performed under a transition catalyst, the Suzuki coupling reaction may use a Pd(0) complex or a Pd(II) salt, and a preferred Pd(0) complex may contain one or more phosphine ligands such as Pd(Ph₃P)₄. Another preferred phosphine ligand may be tris(ortho-tolyl)phosphine (Pd(o-Tol)₄).

A preferred Pd(II) salt may be palladium acetate, that is, Pd(OAc)₂. The Suzuki coupling may be performed in the presence of a base, for example, sodium carbonate, potassium phosphate, or an organic base, for example, tetraethylammonium carbonate, and Yamamoto polymerization may use a Ni(0) complex, for example, bis(1,5-cyclooctadienyl nickel (0).

A reaction time in the method for preparing a micrococcin compound of Chemical Formula 2 according to an exemplary embodiment may vary depending on reaction materials, the type of solvent, and the amount of solvent, and as an example, the reaction is completed after confirming that the starting material is completely consumed by TLC and the like. When the reaction is completed, the solvent is distilled under reduced pressure, and then a target material may be separated and purified by a common method such as column chromatography.

In a specific example, the micrococcin compound of Chemical Formula 2 may be separated from a natural product.

In a specific example, the micrococcin compound of Chemical Formula 2-1-A may be prepared by:
1) producing an expression vector including BcTclE, BcTclI, BcTclJ, BcTclK, BcTclL, BcTclM, BcTclN, and BcTclP genes; and
2) transforming Bacillus subtilis with the expression vector of 1).

In a specific example, the micrococcin compound of Chemical Formula 2-1-B may be prepared by:
1) producing an expression vector including BcTclE, BcTclO, BcTclI, BcTclJ, BcTclK, BcTclL, BcTclM, BcTclN, and BcTclP genes; and
2) transforming Bacillus subtilis with the expression vector of 1).

In a specific example, the micrococcin compound of Chemical Formula 2-1-C may be prepared by:
1) producing an expression vector including BcTclE, BcTclD, BcTclC, BcTclI, BcTclJ, BcTclK, BcTclL, BcTclM, BcTclN, and BcTclP genes; and
2) transforming Bacillus subtilis with the expression vector of 1).

A step of fermenting and culturing the Bacillus subtilis transformed in 2) to produce and secrete each of the micrococcin compounds may be further included, but is not limited thereto.

In the method for preparing a micrococcin-based carboxylic acid compound of the present invention, a carboxylic acid group is introduced to the micrococcin-based compound, whereby it is very easy to introduce various functional groups, and thus, various micrococcin derivatives are possible.

In addition, the present invention provides a biosynthesis method of a micrococcin compound which is a starting material for preparing a micrococcin-based carboxylic acid compound.

The biosynthesis method of a micrococcin compound according to the present invention, which is a method for preparing a micrococcin compound of the following Chemical Formula 2-2, includes:
1) producing an expression vector including BcTclE, BcTclI, BcTclJ, BcTclK, BcTclL, BcTclM, BcTclN, and BcTclP genes; and
2) transforming Bacillus subtilis with the expression vector of 1): 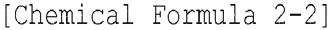 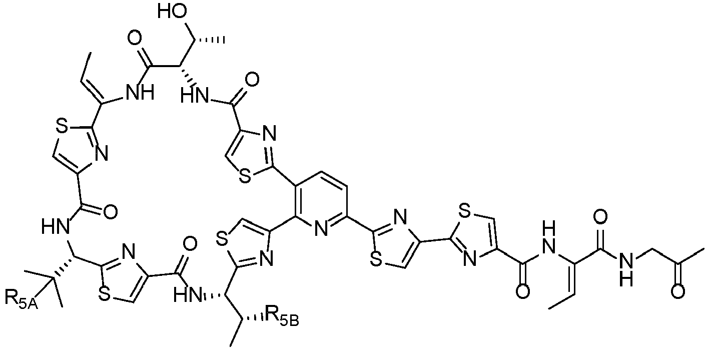 wherein
   R_{5A} is hydrogen or hydroxy, and R_{5B} is hydroxy or methoxy.

In an exemplary embodiment, the expression vector may further include one or more of BcTclO, BcTclD, and BcTclC genes.

In an exemplary embodiment, the expression vector may further include a BcTclO genes.

In a specific example, the expression vector may further include BcTclD and BcTclC genes.

The micrococcin compound may be biosynthesized in a transformat which is transformed with the vector to which the genes of the present invention are introduced, the micrococcin compound which is a target material may be efficiently biosynthesized in Bacillus subtilis cells transformed with the vector including specific genes, and various substituents may be introduced to a macrocycle by further introducing a useful gene.

In addition, the present invention provides a micrococcin-based carboxylic acid compound which is useful as an intermediate for preparing a micrococcin derivative which may have an excellent antibiotic effect represented by Chemical Formula 1.

In addition, the present invention provides a method for preparing a micrococcin derivative useful as an antibiotic using the micrococcin-based carboxylic acid compound prepared above as an intermediate.

The method for preparing a micrococcin derivative of the present invention includes:
reacting a micrococcin compound of the following Chemical Formula 2 with a metal hydroxide to prepare a micrococcin-based carboxylic acid compound of the following Chemical Formula 1; and
preparing a micrococcin derivative of the following Chemical Formula 3 from the micrococcin-based carboxylic acid compound of the following Chemical Formula 1:
   [175] 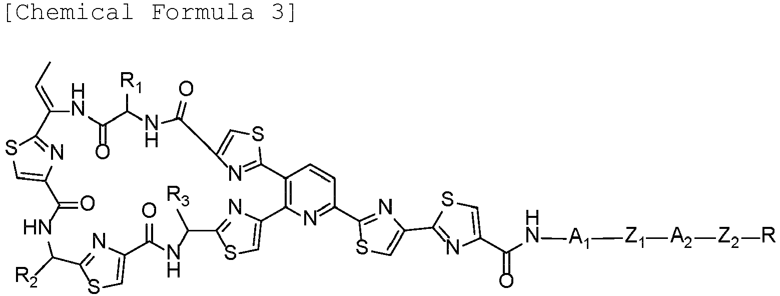 [176] 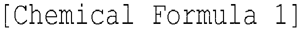 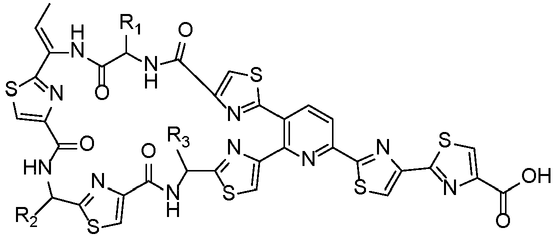 [178] [179] 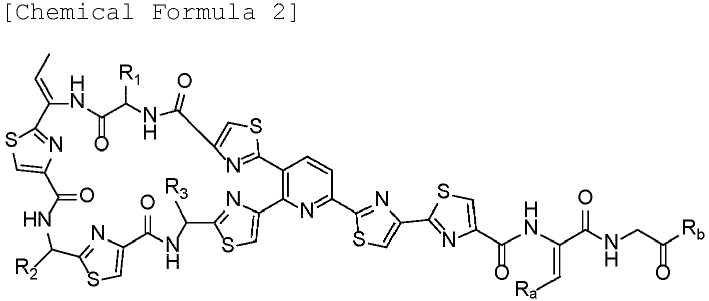 wherein
   R₁ to R₃ are independently of one another hydrogen, C1-C10 alkyl, C3-C10 cycloalkyl, C6-C12 aryl, or C3-C10 heteroaryl;
   the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl;
   Rₐ is hydrogen, C1-C10 alkyl, C2-C10 alkenyl, or C1-C10 alkoxyC1-C10 alkyl;
   R_{b} is hydrogen or C1-C10 alkyl;
   Z₁ and Z₂ are independently of each other a single bond, -CONR₁₁-, -NR₁₂CO-, -COO-, -OCO-, -CR₁₃R₁₄-, -NR₁₅COO-, -NR₁₆-, -S-, -O-, -SO₂-, or -OCONR₁₇-, in which R₁₁ to R₁₇ are independently of one another hydrogen, hydroxy, C1-C10 alkyl, carboxylC1-C10 alkyl, or C1-C10 alkoxycarbonylC1-C10 alkyl;
   A₁ is 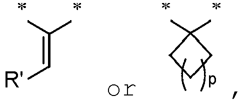 in which R' is hydrogen, C1-C10 alkyl, C2-C10 alkenyl, or C1-C10 alkoxyC1-C10 alkyl, and p is an integer of 0 to 4;
   A₂ is a single bond, C1-C10 alkylene, C3-C10 cycloalkylene, C3-C10 heterocycloalkylene, C6-C20 arylene, or C6-C20 heteroarylene; and
   R is hydrogen, halogen, amino, hydroxy, -B(OH)₂, substituted or unsubstituted haloC1-C10 alkyl, substituted or unsubstituted C1-C10 alkyl, substituted or unsubstituted C2-C10 alkenyl, substituted or unsubstituted C3-C10 cycloalkyl, substituted or unsubstituted C3-C10 heterocycloalkyl, substituted or unsubstituted C6-C20 aryl, or substituted or unsubstituted C3-C20 heteroaryl.

In an exemplary embodiment, the micrococcin-based carboxylic acid compound of Chemical Formula 1 may produce the micrococcin derivative of Chemical Formula 3 by a reaction with an amine compound of the following Chemical Formula 4:

[Chemical Formula 4] H₂N-A₁-Z₁-A₂-Z₂-R

wherein A₁, A₂, Z₁, Z₂, and R are as defined in Chemical Formula 3.

The micrococcin derivative of Chemical Formula 3 according to an exemplary embodiment is effective for killing and inhibiting various bacteria and has a very good effect as an antibiotic.

In Chemical Formula 3 according to an exemplary embodiment, Z₁ may be a single bond, -CONR₁₁-, -NR₁₂CO-, or - COO-; Z₂ may be a single bond, -CONR₁₁-, -NR₁₂CO-, -NR₁₅COO-, -NR₁₆-, -S-, or -OCONR₁₇-; and R₁₁, R₁₂, and R₁₅ to R₁₇ may be independently of one another hydrogen, hydroxy, C1-C10 alkyl, C1-C10 alkoxycarbonylC1-C10 alkyl, or carboxylC1-C10 alkyl.

In Chemical Formula 3 according to an exemplary embodiment, R may be hydrogen, amino, -B(OH)₂, C3-C10 heterocycloalkyl, or C3-C20 heteroaryl, and the heterocycloalkyl or heteroaryl of the alkyl may be further substituted by one or more selected from the group consisting of halogen, amino, nitro, hydroxy, a carboxylic acid group, -B(OH)₂, C1-C10 alkylcarbonyl, C1-C10 alkoxycarbonyl, C2-C10 alkenyl, C1-C10 alkyl, haloC1-C10 alkyl, C3-C10 heterocyclocarbonyl, allylamino, C1-C10 alkylsulfonyl, aminosulfonyl, aminoC1-C10 alkyl, hydroxyC1-C10 alkyl, dihydroxyC1-C10 alkyl, cyanoC1-C10 alkyl, C1-C10 alkylamino, diC1-C10 alkylamino, C6-C20 arylamino, diC6-C20 arylamino, C3-C20 heteroaryl, haloC6-C20 aryl, haloC1-C10 alkylC6-C20 aryl, C6-C20 aryl, C3-C10 cycloalkyl, C3-C10 cycloalkylcarbonyl, C1-C10 alkoxycarbonylC1-C10 alkyl, and carboxylic acid C1-C10 alkyl.

Preferably, in Chemical Formula 3 according to an exemplary embodiment, Z₁ and Z₂ may be independently of each other a single bond, -CONR₁₁-, -COO- or -NR₁₆-; and R₁₁ and R₁₆ may be independently of each other hydrogen.

More preferably, in Chemical Formula 3 according to an exemplary embodiment, Z₁ may be a single bond, -CONR₁₁-, or -COO-; Z₂ may be a single bond, -CONR₁₁-, or -NR₁₆-; and R₁₁ and R₁₆ may be independently of each other hydrogen.

Preferably, in Chemical Formula 3 according to an exemplary embodiment, A₁ may be 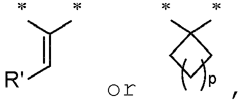 in which R' is C1-C10 alkyl, and p is an integer of 0 to 2; A₂ may be a single bond or C1-C10 alkylene; R may be hydrogen, amino, or -B(OH)₂, or any one selected from the following structures: 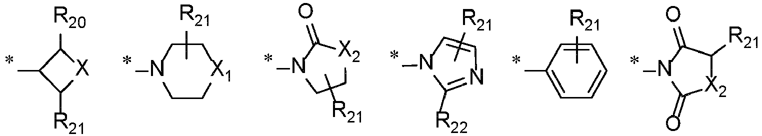 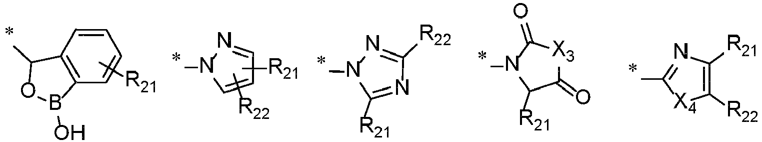 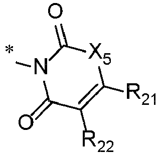 wherein
X is O or S;
X₁ is NR₃₁, O, S, or SO₂;
X₂ to X₅ are independently of one another NR₃₂, O, or S; and
R₂₀ to R₂₂, R₃₁, and R₃₂ are independently of one another hydrogen, halogen, nitro, C1-C10 alkylcarbonyl, C1-C10 alkoxycarbonyl, C1-C10 alkyl, C1-C10 alkylsulfonyl, aminosulfonyl, hydroxyC1-C10 alkyl, dihydroxyC1-C10 alkyl, cyanoC1-C10 alkyl, C3-C10 cycloalkyl, C1-C10 alkoxycarbonylC1-C10 alkyl, or carboxylC1-C10 alkyl.

More preferably, in an exemplary embodiment, R may be hydrogen, amino, or -B(OH)₂, or any one selected from the following structures: 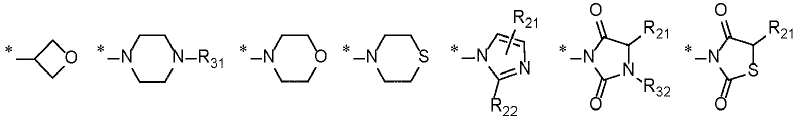 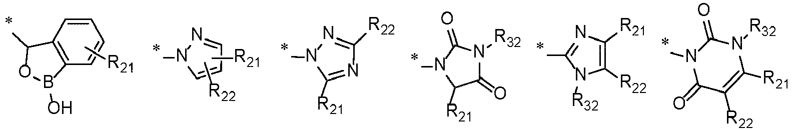 wherein
R₂₁ and R₂₂ are independently of each other hydrogen, halogen, nitro, C1-C10 alkylcarbonyl, C1-C10 alkoxycarbonyl, C1-C10 alkyl, C1-C10 alkylsulfonyl, aminosulfonyl, hydroxyC1-C10 alkyl, dihydroxyC1-C10 alkyl, cyanoC1-C10 alkyl, C3-C10 cycloalkyl, C1-C10 alkoxycarbonylC1-C10 alkyl, or carboxylic acid C1-C10 alkyl; and
R₃₁ and R₃₂ are independently of each other hydrogen, C1-C10 alkylcarbonyl, C1-C10 alkoxycarbonyl, C1-C10 alkyl, C1-C10 alkylsulfonyl, aminosulfonyl, hydroxyC1-C10 alkyl, dihydroxyC1-C10 alkyl, cyanoC1-C10 alkyl, C3-C10 cycloalkyl, C1-C10 alkoxycarbonylC1-C10 alkyl, or carboxylic acid C1-C10 alkyl.

More preferably, R₂₁ and R₂₂ may be independently of each other hydrogen, halogen, nitro, C1-C10 alkyl, or C3-C10 cycloalkyl; and R₃₁ and R₃₂ are independently of each other hydrogen, C1-C10 alkyl, C1-C10 alkylsulfonyl, aminosulfonyl, hydroxyC1-C10 alkyl, dihydroxyC1-C10 alkyl, cyanoC1-C10 alkyl, C3-C10 cycloalkyl, C1-C10 alkoxycarbonylC1-C10 alkyl, or carboxylic acid C1-C10 alkyl.

The micrococcin derivative of Chemical Formula 3 according to an exemplary embodiment may be selected from the following structures, but is not limited thereto:

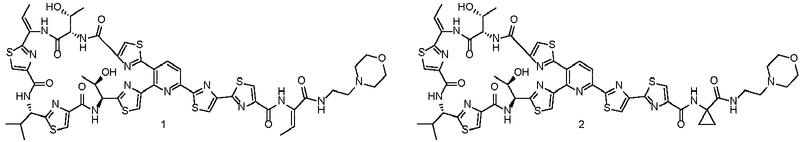

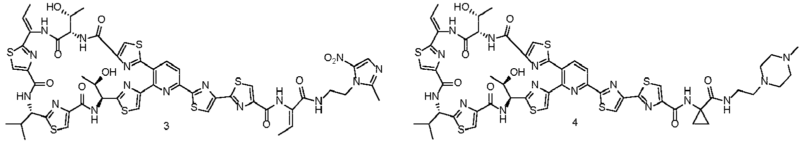

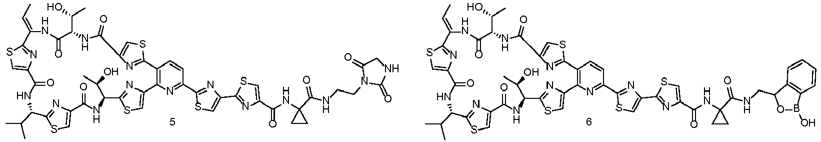

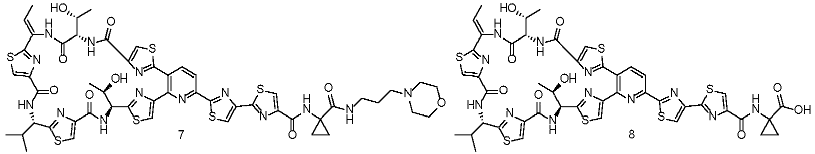

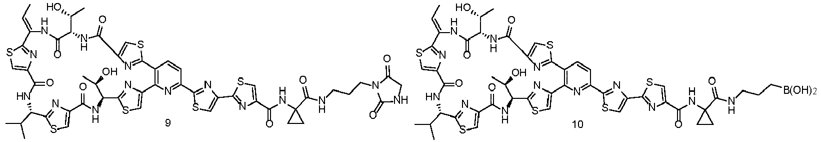

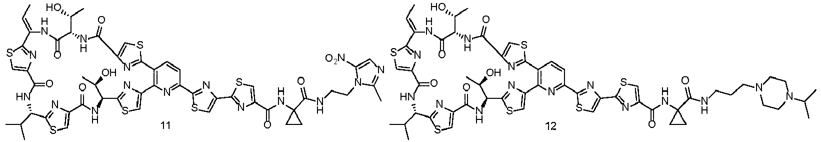

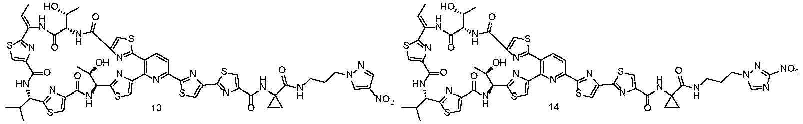

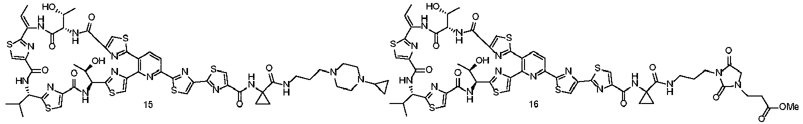

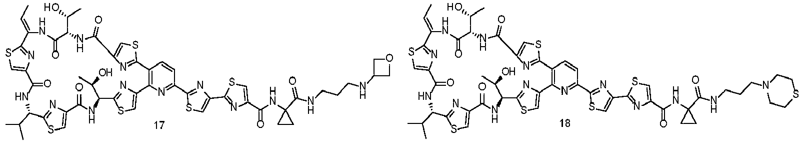

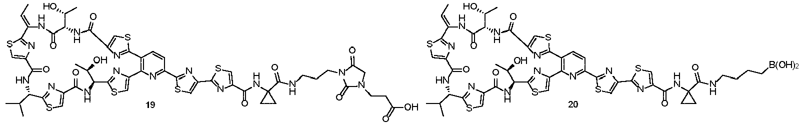

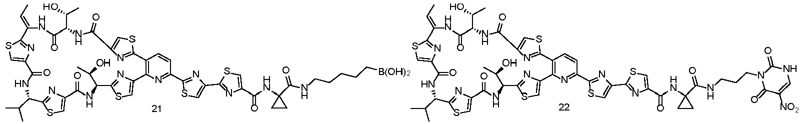

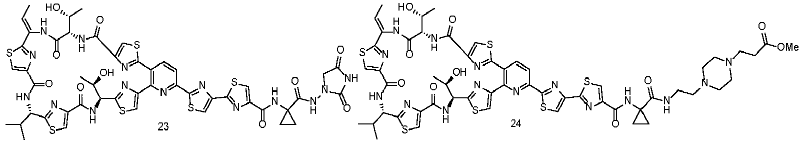

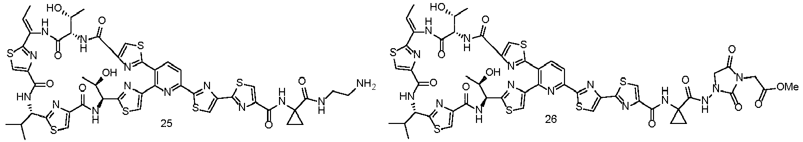

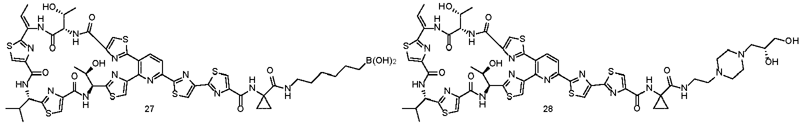

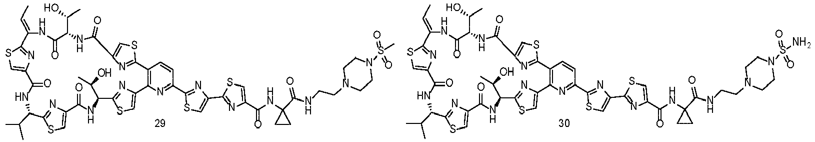

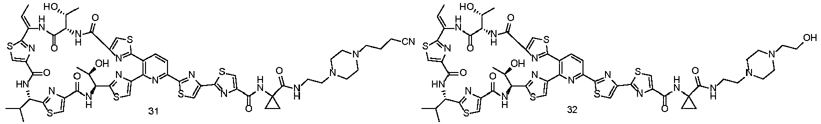

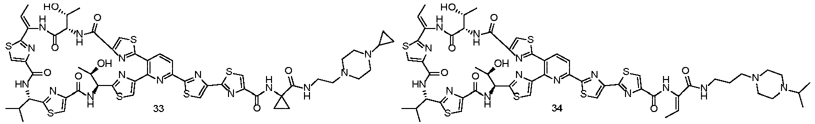

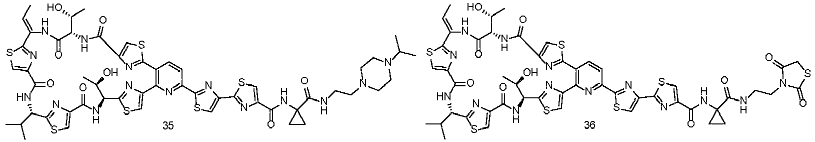

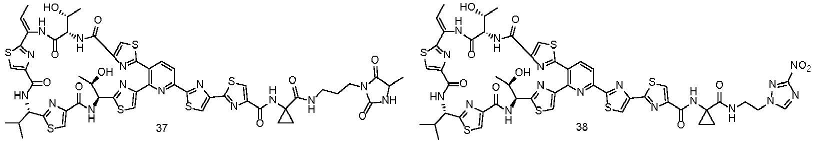

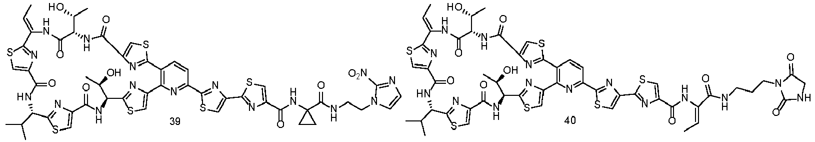

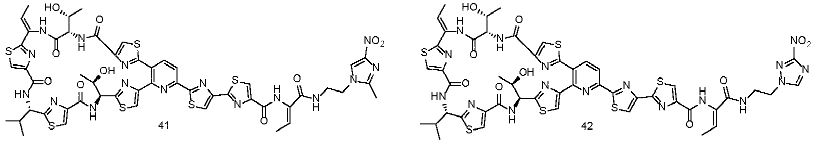

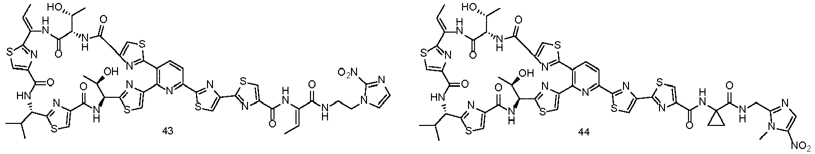

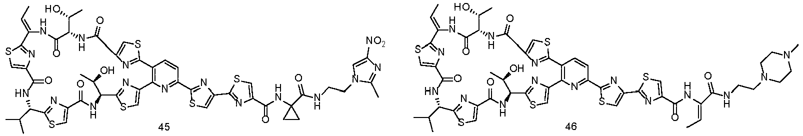

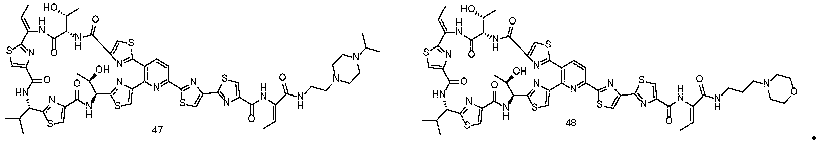

An effect of the micrococcin derivative of Chemical Formula 3 according to an exemplary embodiment as an antibiotic is described in detail in KR 10-2022-0068934 A.

Hereinafter, the method for preparing a micrococcin compound of the present invention will be described with specific examples, but the present invention is not defined by the specific examples.

### [Preparation Example 1] Preparation of Compound A1

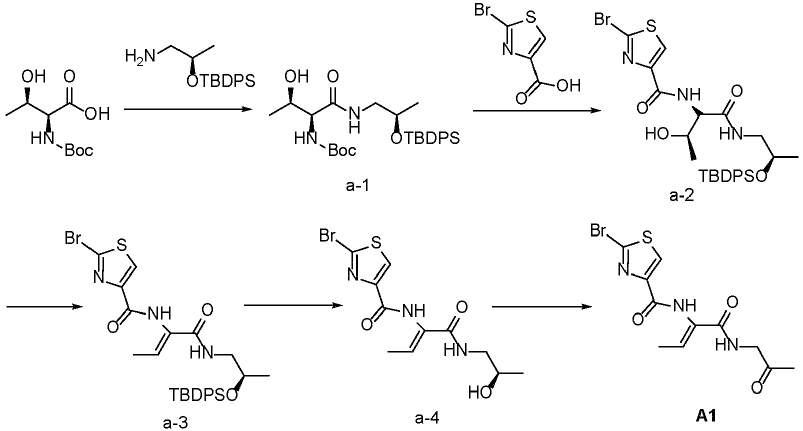

### Step 1: Preparation of Compound a-1

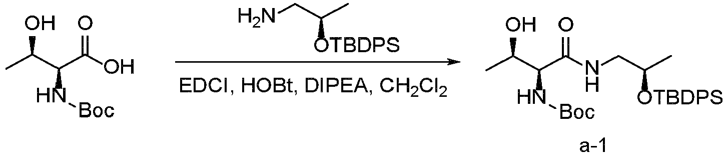

*N*-Boc-*L*-threonine (2 g, 9.2 mmol) and (*R*)-2-((*tert-*butyldiphenylsilyl)oxy)propane-1-amine (3.46 g, 11 mmol, 1.2 eq) were dissolved in dichloromethane (40 mL), 1-ethyl-3-(3-dimethylaminopropyl)carbodiimide (EDCI, 2.1 g, 11 mmol, 1.2 eq), hydroxybenzotriazole (HOBt, 1.48 g, 11 mmol, 1.2 eq), and diisopropylethylamine (DIPEA, 3.2 ml, 18.4 mmol, 2 eq) were added dropwise, and stirring was performed at room temperature for 2 hours. Dilution with dichloromethane and washing with water were performed. An organic layer was extracted, dehydration with anhydrous sodium sulfate was performed, and separation was performed using column chromatography with a 20%-40% ethyl acetate/hexane solution to obtain white solid Compound **a-1** (2.80 g, , 60%).

¹H NMR (600 MHz, CDCl₃) : δ 7.69 (m, 4H), 7.49 - 7.44 (m, 2H), 7.43 - 7.39 (m, 4H), 6.89 (brs, 1H), 5.47 (d, *J* = 7.9 Hz, 1H), 4.34 (qd, *J=* 6.4, 2.0 Hz, 1H), 4.05 - 3.97 (m, 2H), 3.34 - 3.30 (m, 1H), 3.24 (dt, *J* = 13.5, 4.5 Hz, 1H), 2.87 (brs, 1H), 1.48 (s, 9H), 1.18 (d, *J* = 6.4 Hz, 3H), 1.10 (s, 9H), 1.06 (d, *J* = 6.2 Hz, 3H); LCMS: 515.2 (M+H⁺) for C₂₈H₄₃N₂O₅Si.

### Step 2: Preparation of Compound a-2

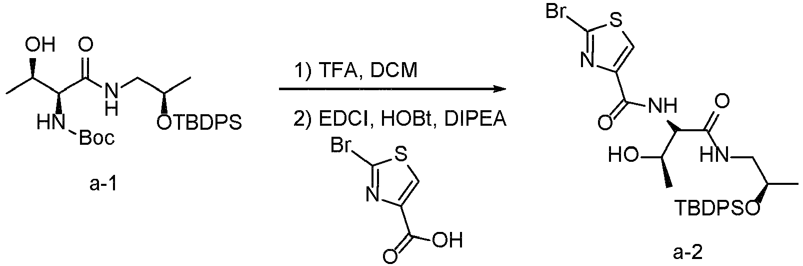

Compound **a-1** (4.50 g, 8.8 mmol, 1.1 eq) was dissolved in dichloromethane (40 mL) / trifluoroacetic acid (40 mL), stirring was performed at room temperature for 1 hour, and concentration under reduced pressure was performed. The obtained compound was dissolved in dichloromethane (20 mL), 2-bromothiazole-4-carboxylic acid (1.66 g, 8 mmol), 1-ethyl-3-(3-dimethylaminopropyl)carbodiimide (1.68 g, 8.8 mmol, 1.1 eq), hydroxybenzotriazole (1.19 g, 8.8 mmol, 1.1 eq), and diisopropylethylamine (4.2 mL, 24 mmol, 3 eq) were added dropwise, and then stirring was performed at room temperature for 12 hours. Dilution with dichloromethane and then washing with water were performed. An organic layer was extracted, dehydration with anhydrous sodium sulfate was performed, and separation was performed using column chromatography with a 20%-50% ethyl acetate/hexane solution to obtain yellow solid Compound **a-2** (3.00 g, 63%).

¹H NMR (600 MHz, CDCl₃) δ 8.08 (s, 1H), 8.01 (d, *J* = 8.4 Hz, 1H), 7.69 - 7.65 (m, 4H), 7.48 - 7.44 (m, 2H), 7.42 - 7.37 (m, 4H), 6.92 - 6.90 (m, 1H), 4.44 (qd, *J =* 6.5, 1.9 Hz, 1H), 4.39 (dd, *J=* 8.4, 1.9 Hz, 1H), 4.02 - 3.97 (m, 1H), 3.55 (brs, 1H), 3.29 (app.t, *J* = 5.3 Hz, 2H), 1.22 (d, *J* = 6.5 Hz, 3H), 1.05 (s, 9H), 1.03 (d, *J* = 6.5 Hz, 3H); LCMS: 604.1 (M+H⁺) for C₂₇H₃₅BrN₃O₄SSi.

### Step 3: Preparation of Compound a-3

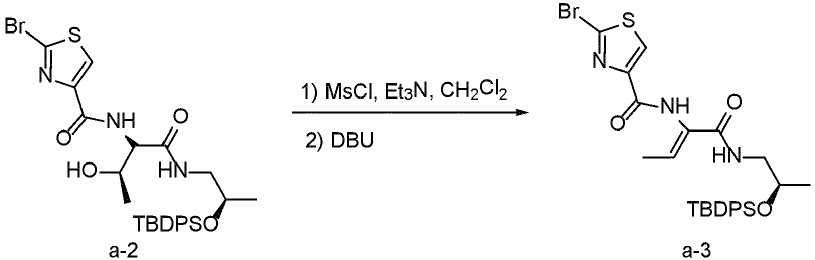

Compound **a-2** (3 g, 5 mmol) was dissolved in dichloromethane (40 mL), methanesulfonyl chloride (MsCl, 1.15 mL, 14.9 mmol, 3 eq) and triethylamine (2.1 mL, 14.9 mmol, 3 eq) were added dropwise, and stirring was performed at room temperature for 1 hour. Then, 1,8-diazabicyclo[5,4,0]undec-7-ene (DBU, 11.2 ml, 74.8 mmol, 15 eq) was added and stirring was performed at room temperature for 2 hours. After confirming reaction completion, dilution with dichloromethane was performed, and supersaturated ammonium chloride was added to wash. An organic layer was extracted, dehydration with anhydrous sodium sulfate was performed, and separation was performed using column chromatography with a 30%-50% ethyl acetate/hexane solution to obtain white solid Compound **a-3** (2.2 g, 75%).

¹H NMR (600 MHz, CDCl₃) : δ 8.32 (s, 1H), 8.12 (s, 1H), 7.66 - 7.64 (m, 4H), 7.48 - 7.41 (m, 2H), 7.40 - 7.37 (m, 4H), 6.53 (q, *J* = 7.1 Hz, 1H), 6.39 (app.t, *J* = 5.3 Hz, 1H), 4.05 - 4.01 (m, 1H), 3.41 (ddd, *J* = 13.6, 5.4, 3.6 Hz, 1H), 3.33 (dt, *J* = 13.6, 5.9 Hz, 1H), 1.79 (d, *J* = 7.1 Hz, 3H), 1.10 (d, *J* = 6.2 Hz, 3H), 0.99 (s, 9H); LCMS: 586.1 (M+H⁺) for C₂₇H₃₃BrN₃O₃SSi.

### Step 4: Preparation of Compound a-4

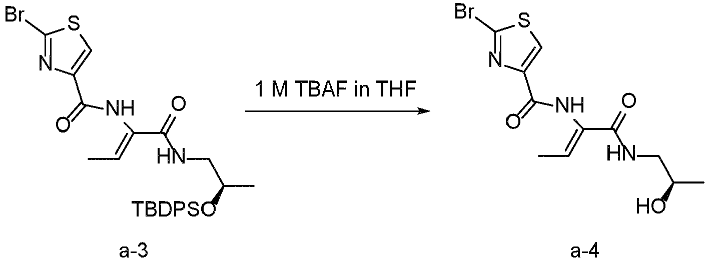

Compound **a-3** (2.2 g, 3.74 mmol) was dissolved in tetrahydrofuran (30 mL), 1 M tetra-n-butylammonium fluoride (TBAF, 6.74 mL, 6.74 mmol, 1.8 eq) was added dropwise, and then stirring was performed at room temperature for 30 minutes. Dilution with ethyl acetate was performed, and supersaturated ammonium chloride was added to wash. An organic layer was extracted, dehydration with anhydrous sodium sulfate was performed, and separation was performed using column chromatography with a (2%-5% methanol: 98%-95% dichloromethane) solution to obtain colorless liquid Compound **a-4** (800 mg, 61%).

¹H NMR (600 MHz, CD₃OD) : δ 8.30 (d, *J* = 0.9 Hz, 1H), 6.71 (q, *J* = 7.1 Hz, 1H), 3.94 - 3.85 (m, 1H), 3.30 (dd, *J* = 13.5, 4.7 Hz, 1H), 3.20 (dd, *J* = 13.4, 7.1 Hz, 1H), 1.79 (d, *J* = 7.1 Hz, 3H), 1.16 (d, *J* = 6.3 Hz, 3H) ; LCMS: 348.0 (M+H⁺) for C₁₁H₁₅BrN₃O₃S.

### Step 5: Preparation of Compound A1

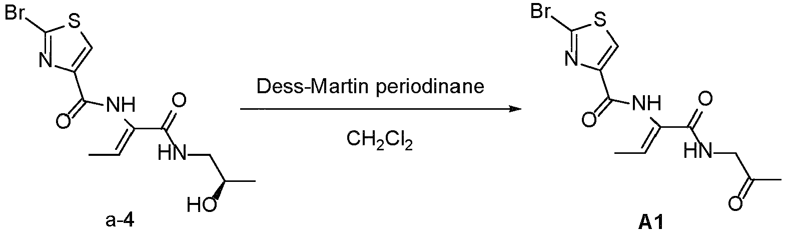

Compound **a-4** (386 mg, 1.12 mmol) was dissolved in dichloromethane (10 mL), and then Dess-Martin periodinane (706 mg, 1.66 mmol, 1.5 eq) was added dropwise. Stirring was performed at room temperature for 2 hours, dilution with dichloromethane was performed, and washing with supersaturated sodium hydrogen carbonate was performed. An organic layer was extracted, dehydration with anhydrous sodium sulfate was performed, and separation was performed using column chromatography with a (2%-5% methanol: 98%-95% dichloromethane) solution to obtain white solid Compound **A1** (356 mg, 89%).

¹H NMR (600 MHz, CD₃OD) : δ 8.30 (s, 1H), 6.77 (q, *J* = 7.1 Hz, 1H), 4.07 (s, 2H), 2.18 (s, 3H), 1.81 (d, *J* = 7.1 Hz, 3H); LCMS: 345.9 (M+H⁺) for C₁₁H₁₃BrN₃O₃S.

### [Example 1] Preparation of Compounds 13 (Micrococcin P2)

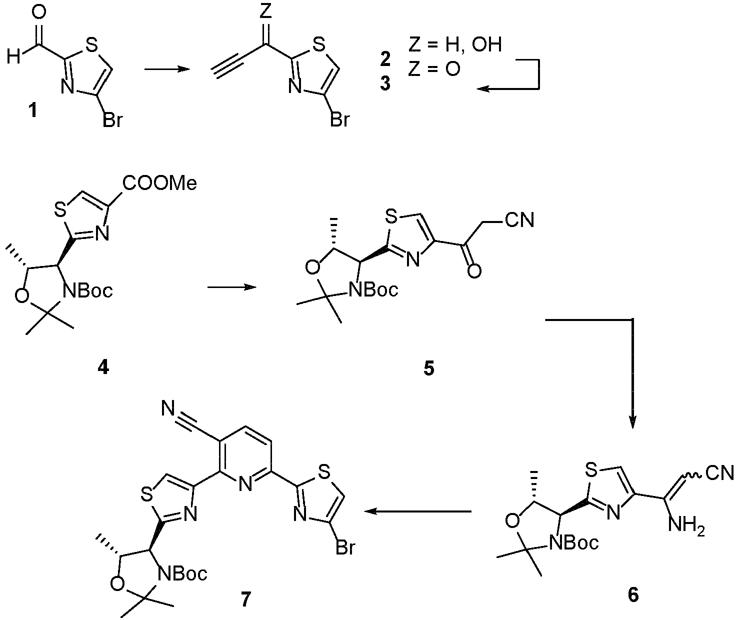

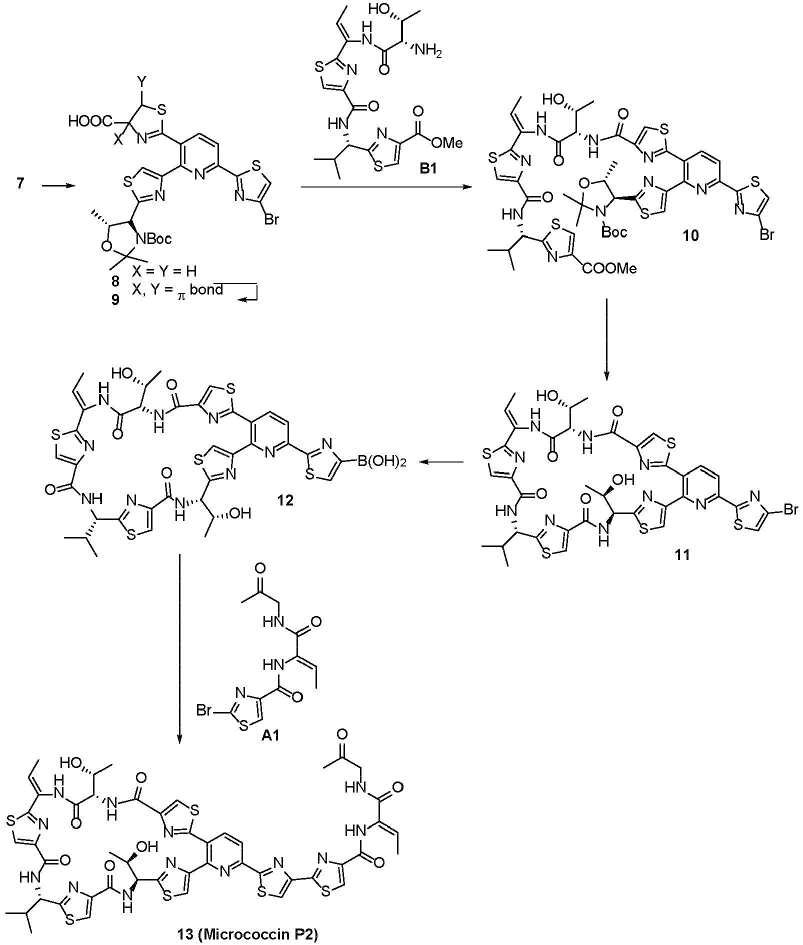

### Step 1: Preparation of Compound 3

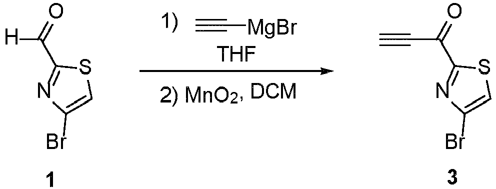

4-Bromo-2-formylthiazole (768 mg, 4 mmol) was dissolved in tetrahydrofuran (10 mL), and an ethynyl magnesium bromide solution (0.5 M in THF, 16 mL, 8 mmol) was slowly added dropwise at 0°C. Stirring was performed at room temperature for 30 minutes, and a supersaturated ammonium chloride solution was added to stop the reaction. Extraction with ethyl acetate, then dehydration with anhydrous sodium sulfate, and concentration under reduced pressure were performed. The obtained residue was separated using column chromatography with a 30% ethyl acetate/70% hexane solution to obtain a white solid compound. The obtained compound (632 mg, 2.9 mmol) was dissolved in dichloromethane (30 mL), manganese dioxide (2.52 g, 29 mmol) was added, and then stirring was performed at room temperature for 2 hours. A manganese residue was filtered using celite, extraction with dichloromethane and dehydration with anhydrous sodium sulfate were performed, and then concentration under reduced pressure was performed to obtain brown solid Compound **3** (527 mg, 61%).

¹H NMR (400 MHz, CDCl₃) δ 7.69 (s, 1H), 3.67 (s, 1H); LCMS: 215.9 (M+H⁺) for C₆H₃BrNOS.

### Step 2: Preparation of Compound 5

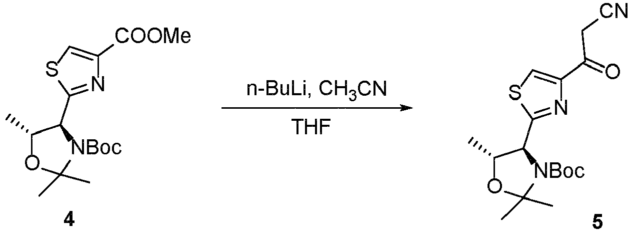

Compound **4** was synthesized by a method disclosed in Org. Lett, 2020, 22, 6, 2365-2370.

Acetonitrile (6.52 mL, 125 mmol, 2 eq) was dissolved in tetrahydrofuran (120 mL), and then a n-butyllithium solution (2.5 M in hexanes, 54.8 mL, 137 mmol, 2.2 eq) was slowly added dropwise at -78°C. Stirring was performed at -78°C for 20 minutes, and then Compound **4** (22.3 g, 62.6 mmol) dissolved in tetrahydrofuran (120 mL) was slowly added dropwise at the same temperature. The temperature was slowly raised to room temperature, the reaction was performed for 1 hour, and hydrochloric acid at a concentration of 1 M was added to stop the reaction. Dilution with ethyl acetate was performed, and hydrochloric acid was further added to perform acidification to pH 3. An organic layer was extracted, dehydration with anhydrous sodium sulfate was performed, and then concentration under reduced pressure was performed to obtain brown solid Compound **5** (22.2 g, 97%).

¹H NMR (400 MHz, CDCl₃) δ 8.29 (s, 1H), 4.71 (d, *J* = 17.7 Hz, 1H), 4.32 - 4.09 (m, 3H), 1.70 (broad peak, 6H), 1.54 - 1.38 (m, 7H), 1.31 - 1.06 (m, 5H); LCMS: 366.1 (M+H⁺) for C₁₇H₂₄N₃O₄S.

### Step 3: Preparation of Compound 6

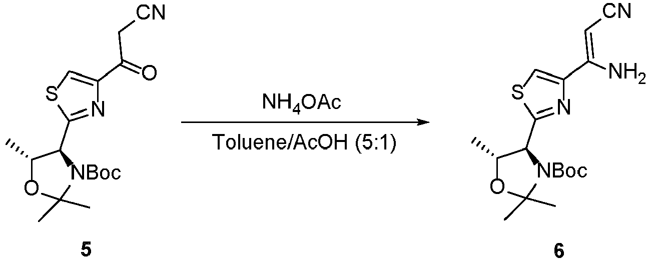

Compound **5** (22.3 g, 61.0 mmol) was dissolved in a mixture of toluene (300 mL) and acetic acid (60 mL), and ammonium acetate (47 g, 610 mmol, 10 eq) was added dropwise. Stirring was performed at 120°C for 1 hour, and slow cooling to room temperature was performed. Dilution with ethyl acetate, and then washing with a supersaturated sodium hydrogen carbonate solution and water were performed. An organic layer was extracted, dehydration with anhydrous sodium sulfate was performed, and then concentration under reduced pressure was performed to obtain yellow oil Compound **6** (21.0 g, 94%).

¹H NMR (400 MHz, CDCl₃) δ 7.60 (s, 1H), 5.59 - 5.51 (m, 2H), 4.81 - 4.50 (m, 2H), 4.21 - 4.10 (m, 1H), 1.67 (s, 6H), 1.48 - 1.36 (m, 6H), 1.27 - 0.96 (m, 6H); LCMS: 365.1 (M+H⁺) for C₁₇H₂₅N₄O₃S.

### Step 4: Preparation of Compound 7

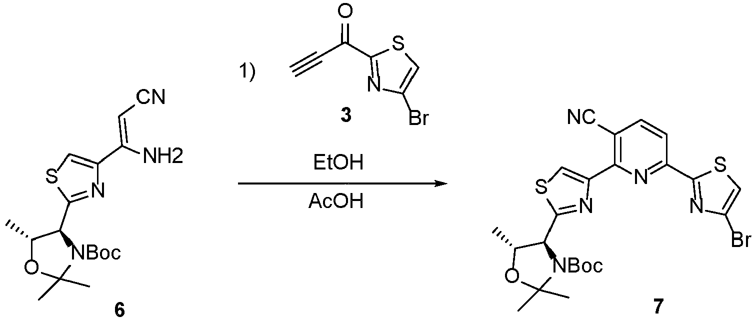

Compound **6** (21.0 g, 57.5 mmol) was dissolved in ethanol (300 ml), Compound **3** (22.9 g, 106.3 mmol, 1.85 eq) was added dropwise, and stirring was performed at 60°C for 90 minutes. After confirming that the starting material disappeared with TLC and LC mass, acetic acid (30% volume, 90 ml) was added, and then the reaction was performed at 90°C for 16 hours. Concentration under reduced pressure was performed to remove ethanol and acetic acid, dilution with dichloromethane was performed, and the reaction was stopped with a supersaturated sodium hydrocarbon carbonate solution. An organic layer was extracted, dehydration was performed with anhydrous sodium sulfate, and then concentration under reduced pressure was performed. Separation using column chromatography with a 5%-15% ethyl acetate/hexane solution was performed to obtain yellow solid Compound **7** (16.5 g, 51%).

¹H NMR (400 MHz, CDCl₃) δ 8.27 (s, 1H), 8.19 (s, 2H), 7.48 (s, 1H), 4.85 (d, *J* = 7.3 Hz, 1H), 4.58 - 4.23 (broad peak, 1H), 1.83 - 1.67 (broad peak, 6H), 1.57 - 1.45 (m, 6H), 1.33 - 1.20 (m, 6H); LCMS: 562.0 (M+H⁺) for C₂₃H₂₅BrN₅O₃S₂.

### Step 5: Preparation of Compound 9

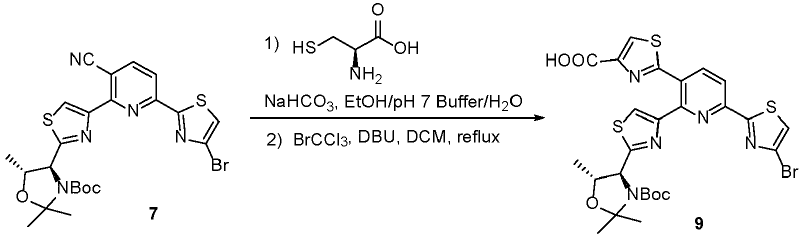

Compound **7** (1.42 g, 2.53 mmol) was dissolved in a mixed solution of ethanol (27 mL), a sodium phosphate buffer solution (pH 7, 9 mL), and water (4.5 mL), and then L-cysteine (3.06 g, 25.3 mmol, 10 eq) was added dropwise. Stirring was performed at 100°C for 24 hours, concentration under reduced pressure was performed to remove ethanol, and dilution with ethyl acetate was performed. Washing with hydrochloric acid at a concentration of 1 M was performed for removing an excessive amount of cysteine, and dehydration was performed with anhydrous sodium sulfate. The solution obtained by extraction was concentrated under reduced pressure to obtain thiazoline of brown oil.

The thiazoline of brown oil (1.68 g, 2.53 mmol) was dissolved in dichloromethane (30 mL), bromotrichloromethane (1.0 mL, 10.12 mmol, 4 eq) was added, and then 1,8-diazabicyclo[5,4,0]undec-7-ene (1.88 mL, 12.65 mmol, 5 eq) was slowly added dropwise. Stirring was performed at 60°C for 4 hours, and then hydrochloric acid at a concentration of 1 M was added to stop the reaction. Extraction with dichloromethane was performed, and then washing with water and brine was performed. The extracted organic layer was dehydrated with anhydrous sodium sulfate, concentrated under reduced pressure, and separated using column chromatography with a 5%-15% methanol/dichloromethane solution to obtain brown solid Compound **9** (1.05 g, 62%).

¹H NMR (400 MHz, CDCl₃) δ 8.35 - 8.05 (m, 3H), 7.89 (bs, 1H), 7.33 (s, 1H), 4.49 - 4.40 (m, 1H), 4.09 - 3.97 (m, 1H), 1.71 - 1.29 (m, 10H), 1.26 - 1.08 (m, 8H); LCMS: 664.0 (M+H⁺) for C₂₆H₂₇BrN₅O₅S₃.

### Step 6: Preparation of Compound 10

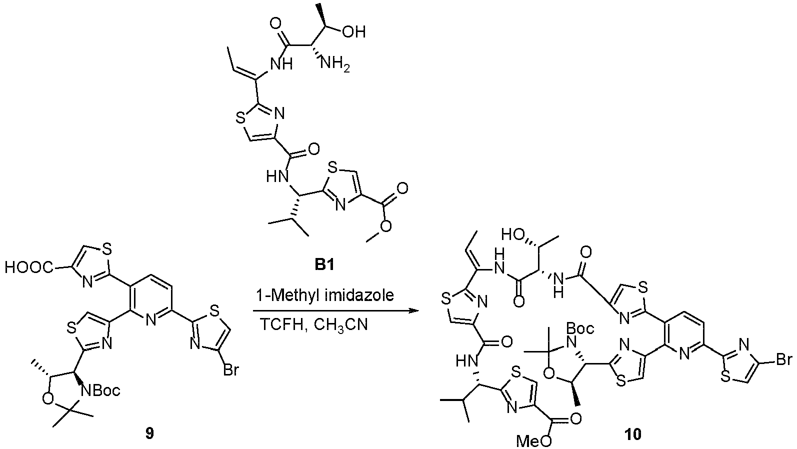

Compound **B1** was prepared according to Org. Lett, 2020, 22, 6, 2365-2370. Compound **9** (1.05 g, 1.57 mmol) was dissolved in acetonitrile (8 mL), Compound **B1** (932 mg, 1.88 mmol), 1-methylimidazole (0.376 mL, 4.71 mmol, 3 eq), and chloro-*N,N,N,N*-tetramethylformamidinium hexafluorophosphate (TCFH, 0.528 g, 1.88 mmol, 1.2 eq) were added, and stirring was performed at room temperature for 30 minutes. The solution was diluted with ethyl acetate and washed with water, an organic layer was dehydrated using anhydrous sodium sulfate, and concentration under reduced pressure was performed. The concentrated solution was separated using column chromatography with a (40%-90 % ethyl acetate: 60%-10% hexane) solution to obtain white solid Compound **10** (1.05 g, 60%).

¹H NMR (400 MHz, CDCl₃) δ 8.69 (s, 1H), 8.44 (d, *J* = 9.5 Hz, 1H), 8.35 - 8.24 (m, 4H), 8.24 - 8.17 (m, 1H), 8.06 (s, 1H), 7.96 (s, 1H), 7.94 - 7.87 (m, 1H), 6.47 (q, 7.1 Hz, 1H), 5.41 - 5.31 (m, 1H), 4.88 (d, *J* = 5.3 Hz, 1H), 4.78 (bs, 1H), 4.68 - 4.58 (m, 1H), 4.58 - 4.46 (m, 1H), 4.16 - 3.98 (m, 1H), 3.86 (s, 3H), 2.49 - 2.45 (m, 1H), 1.87 (d, *J* = 7.1 Hz, 3H), 1.69 - 1.48 (m, 6H), 1.45 - 1.32 (m,3H), 1.31 - 1.08 (m, 12H), 1.03 (d, *J* = 6.8 Hz, 3H), 0.95 (d, *J* = 6.7 Hz, 3H); LCMS: 1127.1 (M+H⁺) for C₄₆H₅₂BrN₁₀O₉S₅.

### Step 7: Preparation of Compound 11

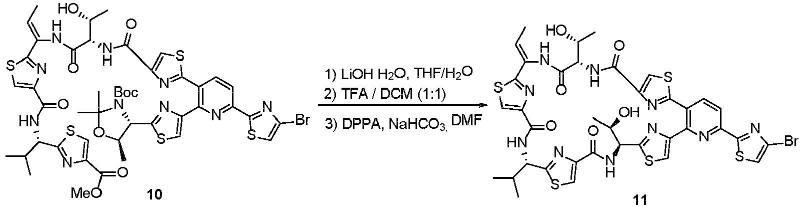

Compound **10** (200 mg, 0.177 mmol) was added to a mixed solvent of tetrahydrofuran (1 mL)/ water (1 mL), lithium hydroxide monohydrate (22 mg, 0.531 mmol, 3 eq) was added, and stirring was performed at room temperature for 2 hours. The solution was diluted with dichloromethane, washed with a 1 M hydrochloric acid solution, dehydrated using anhydrous sodium sulfate, and concentrated under reduced pressure to obtain carboxylic acid. The concentrated solution was added to a mixed solvent of dichloromethane (1 mL)/ trifluoroacetic acid (1 mL), stirring was performed at room temperature for 30 minutes, and then concentration under reduced pressure was performed to obtain a trifluoroacetic acid salt. The concentrated solution was diluted with DMF (1 mL), diphenylphosphoryl azide (0.038 mL, 0.177 mmol, 1 eq) and sodium hydrogen carbonate (0.045 mL, 0.531 mmol, 3 eq) were added, and stirring was performed at room temperature for 12 hours. The solution was diluted with dichloromethane, an organic layer was washed with water three times, dehydrated with anhydrous sodium sulfate, concentrated under reduced pressure, and then separated using column chromatography with a (2%-5% methanol: 98%-95% dichloromethane) solution to synthesize yellow solid Compound **11** (96 mg, 56%).

¹H NMR (400 MHz, CDCl₃) δ 8.77 (s, 1H), 8.39 (d, *J* = 9.6 Hz, 1H), 8.22 - 8.18 (m, 2H), 8.16 - 8.09 (m, 3H), 8.01 - 7.92 (m, 3H), 7.40 (s, 1H), 6.39 (q, *J* = 7.0 Hz, 1H), 5.27 - 5.18 (m, 2H), 4.92 (d, *J* = 7.8 Hz, 1H), 4.67 (s, 1H), 4.47 - 4.33 (m, 1H), 2.95 (bs, 1H), 2.48 (dq, *J* = 13.4, 6.6 Hz, 1H), 2.22 (bs, 1H), 1.81 (d, *J* = 7.0 Hz, 3H), 1.57 (d, *J* = 5.2 Hz, 3H), 1.19 (d, *J* = 6.5 Hz, 3H), 1.14 (d, *J* = 6.7 Hz, 3H), 0.95 (d, *J* = 6.6 Hz, 3H); LCMS: 955.0 (M+H⁺) for C₃₇H₃₆BrN₁₀O₆S₅.

### Step 8: Preparation of Compound 12

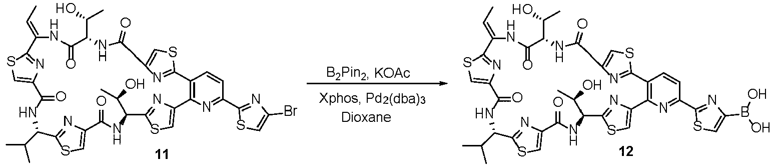

Compound **11** (41 mg, 0.043 mmol) was dissolved in 1,4-dioxane (0.6 mL), and bis(pinacolato)diborone (B₂Pin₂, 14.2 mg, 0.056 mmol, 1.3 eq), potassium acetate (KOAc, 6.7 mg, 0.065 mmol, 1.5 eq), and Xphos (3.1 mg, 15 mol%) were added. After the addition, nitrogen was added for 1 minute, tris(dibenzylideneacetone)dipalladium(0) (Pd₂(dba)₃, 4mg, 10 mol%) was added dropwise. Stirring was performed at 90°C for 3 hours, dilution with dichloromethane was performed, and an organic layer was extracted. The extracted organic layer was washed with water, dehydrated with anhydrous sodium sulfate, and then concentrated under reduced pressure to obtain solid Compound **12** (18 mg, 47 %).

¹H NMR (400 MHz, MeOD) δ 8.40 - 8.21 (m, 4H), 8.13 (s, 1H), 7.93 (s, 1H), 7.76 (s, 1H), 6.61 (q, *J* = 6.9 Hz, 1H), 5.29 - 5.19 (m, 2H), 4.75 (d, *J* = 3.1 Hz, 1H), 4.62 - 4.50 (m, 1H), 4.30 (dd, *J* = 6.3, 2.9 Hz, 1H), 2.64 (dq, *J* = 13.4, 6.7 Hz, 1H), 1.85 (d, *J* = 7.0 Hz, 3H), 1.53 (d, *J* = 6.4 Hz, 3H), 1.19 (d, *J* = 6.3 Hz, 3H), 1.13 (d, *J* = 6.7 Hz, 3H), 0.99 (d, *J* = 6.7 Hz, 3H); LCMS: 921.1 (M+H⁺) for C₃₇H₃₈BN₁₀O₈S₅.

### Step 9: Preparation of Compound 13

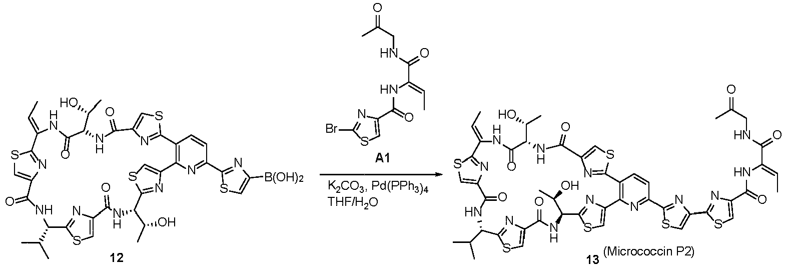

Compound **12** (276 mg, 0.3 mmol) was dissolved in a mixed solvent of tetrahydrofuran (6 mL) and water (1.5 mL). Compound **A1** (155 mg, 0.45 mmol, 1.5 eq) was added dropwise, and nitrogen was supplied for 3 minutes. Thereafter, potassium carbonate (124 mg, 0.9 mmol, 3 eq) and tetrakis(triphenylphosphine) palladium (Pd(PPh₃)₄, 10 mol%, 35 mg) were added, and nitrogen was added again for 1 minute. Stirring was performed at 38°C for 3 hours, and then concentration under reduced pressure was performed. The obtained residue was separated using column chromatography with a 0-5% methanol/dichloromethane solution to obtain white solid Compound **13** (179 mg, 55%).

¹H NMR (600 MHz, MeOD) δ 8.40 - 8.31 (m, 4H), 8.27 (s, 1H), 8.22 (s, 1H), 8.13 (s, 1H), 7.96 (s, 1H), 6.82 (q, *J* = 7.1 Hz, 1H), 6.60 (q, *J* = 6.8 Hz, 1H), 5.24 (d, *J* = 2.9 Hz, 1H), 5.22 (d, *J* = 8.8 Hz, 1H), 4.78 (d, *J* = 3.1 Hz, 1H), 4.62 - 4.55 (m, 1H), 4.30 (qd, *J* = 6.3, 2.8 Hz, 1H), 4.10 (d, *J* = 13.0 Hz, 1H), 2.63 (dq, *J* = 13.4, 6.7 Hz, 1H), 2.19 (s, 3H), 2.20 - 2.14 (m, 1H), 1.92 (s, 1H) 1.86 (app. d, *J* = 7.0 Hz, 6H), 1.55 (d, *J* = 6.4 Hz, 3H), 1.18 (d, *J* = 6.3 Hz, 3H), 1.13 (d, *J* = 6.7 Hz, 3H), 0.99 (d, *J* = 6.7 Hz, 3H); LCMS: 1142.2 (M+H⁺) for C₄₈H₄₈N₁₃O₉S₆.

### [Example 2] Preparation of micrococcin compound

### 1) Bacillus cereus genomic DNA separation

For culturing *Bacillus cereus* ATCC 14579 strains, a Luria-Bertani (LB) medium (Becton, Dickinso and Company, New Jersey, USA) was used to inoculate a single colony formed in a Luria-Bertani agar medium into 5 ml of an LB broth, which was cultured at 30°C for 3 days. A genomic DNA mini kit (genomic DNA extraction kit; promega, WI, USA) was used to separate genome DNA from the strains separated thereby.

### 2) Vector production

A gene cluster which biosynthesizes Thiocillin consists of 24 biosynthesis-related genes spanning 22 kb. A vector which may synthesize Micrococcin P2 using 8 genes among them was produced, and was expressed in Bacillus subtilis 168 to intend to secure a material.

The biosynthesis genes used in vector production were BcTclE, BcTclI, BcTclJ, BcTclK, BcTclL, BcTclM, BcTclN, and BcTclP. In the case of BcTclIJKLMNP, gene cloning was performed on a xylose inducible promotor, and in the case of BcTclE also, four BcTclE genes were connected on the same xylose inducible promotor to secure a recombinant plasmid.

As a vector for Bacillus transformation, a pUC19 was prepared after modification. pUC19 was produced using amyE, cat, ThrC, base sequence introduced from a pDG1661 vector in order to be used in Bacillus transformation. In Vector No. 1, a vector designed so that transformed Bacillus subtilis was integrated to amyE locus to have resistance to chloramphenicol was used to insert biosynthesis genes. That is, in Vector No. 1, Micrococcin P2 biosynthesis genes (BcTclE,J,K,L,M,N,P) were cloned, and in Vector No. 2, a BcTclO gene was further inserted into Vector No. 1. The Vector No. 3 is a vector to which BcTclD and BcTclC vectors were inserted.

Each gene was amplified from DNA by a common polymerase chain reaction (PCR) method and gene cloning was performed using Gibson assembly (NEB, England) or ligase. The vector used therein was a vector modified so that Bacillus subtilis is transformed with pUC19, and the primer used for cloning and vector modification was as described in the following Tables 1 to 3.

**[Table 1]**

| Vector No. 1 | | | |
|---|---|---|---|
| No. | Name | Primer sequence | Template |
| 1 | amyE-F | GTGCACCATATGTTTGCAAAACGATTCAAAACC | pDG1661 |
| 2 | amyE-R | TCGAATTCCGTATCATGCGACTCTACC | pDG1661 |
| 3 | cat-amyE-F | CGACCTGCAGGCATGCAAGCTTGCACCCATTAGTTCAACAAAC | pDG1661 |
| 4 | cat-amyE-R | ATGACCATGATTACGCCAAGCTATCAATGGGGAAGAGAACCGC | pDG1661 |
| 5 | xylR-CRE-F | ACGGAATTCGAGCTCGGTACCGAACTCCTTTTTCATATGAGAAGG | gDNA |
| 6 | xylR-CRE-R | TTCTAGAGGATACCCGGGTACCTTAGTAAACCACTTTGTTTGCGCTTCC | gDNA |
| 7 | BcTclE-F | GGGGCTAGCTTTAAGAAGGAGATATACAT | gDNA |
| 8 | BcTclE-R | GCTCTAGATTAAGTTGTACAACAACTGC | gDNA |
| 9 | BcTclI-F | GTTTACTAAGGTACCCGGGATCCGTTTGTTCCCTTTATAGAAAGG | gDNA |
| 10 | BcTclJ-R | TCAGTGGTGGTGGTGGTGGTGCTCGAGTTATGGGAAAGGATGAGGAGTTA C | gDNA |
| 11 | BcTclK-F | TCCTCATCCTTTCCCATAACATTTTTTATGAAAGATGG | gDNA |
| 12 | BcTclL-R | TCAGTGGTGGTGGTGGTGGTGCTCGAGTCATCCCTTTCTACTCTTATACA ATG | gDNA |
| 13 | BcTclM-F | ATAAGAGTAGAAAGGGATGACTTAAATGGAGCAGTATC | gDNA |
| 14 | BcTclN-R | TCAGTGGTGGTGGTGGTGGTGCTCGAGTCATACATAAAATTGTTCTCCTT TAGTT | gDNA |
| 15 | BcTclP-F | TAAAGGAGAACAATTTTATGTATGACGGAAAACGGGTGAATC | gDNA |
| 16 | BcTclP-R | TCAGTGGTGGTGGTGGTGGTGTCGAGATCTAGTGTAATGTATAACCACC | gDNA |

**[Table 2]**

| Vector No. 2 | | | |
|---|---|---|---|
| No. | Name | Primer sequence | Template |
| 1 | TclO-F | AGGAGAACAATTTTATGTATGACGAATAAAACTAAAGGAGAAC | gDNA |
| 2 | TclO-R | TCAGTGGTGGTGGTGGTGGTGCTCGAGCTAACGTTTTATTCCATTCAC | gDNA |

**[Table 3]**

| Vector No. 3 | | | |
|---|---|---|---|
| No. | Name | Primer sequence | Template |
| 1 | TclD-F | TGCAGTTGTTGTACAACTTAACAATTAGAGAGGAGTATAC | gDNA |
| 2 | TclC-R | TCAGTGGTGGTGGTGGTGGTGCTCGAGTCAAGAAAGATTAATATTATCTAC | gDNA |

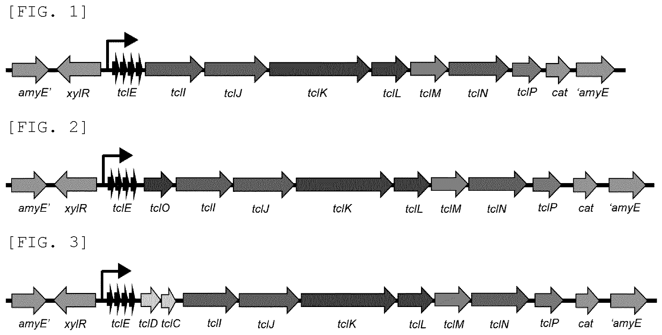

As a host cell for gene cloning, Escherichia coli DH5α (Enzynomics, Korea) was selected, it was confirmed that there were no mutations in genes by sequencing, and then molecule cloning was performed. Thus, a vector which may biosynthesize thiopeptide from Bacillus was produced. Schematic diagrams of genetic recombination plasmid which was newly introduced to pUC19 are shown in FIGS. 1, 2, and 3, and its base sequence is listed in sequence listing.

### 3) Material synthesis and analysis in transformed Bacillus

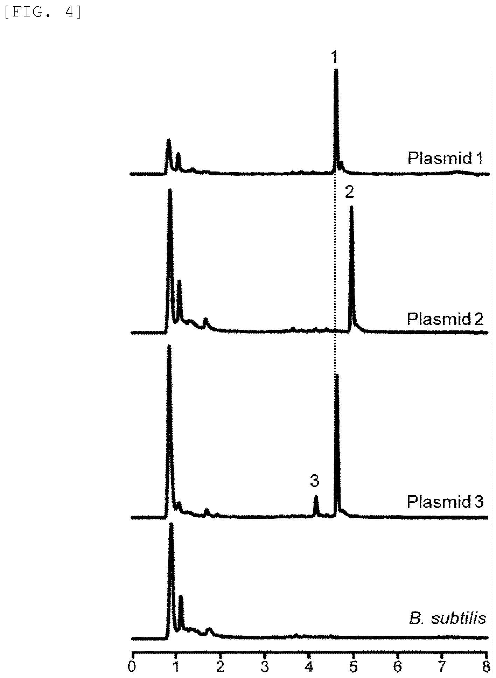

Three plasmids shown in FIGS. 1 to 3 were transformed using commonly known protocol in Bacillus subtilis 168 hosts. To the transformed Bacillus subtilis in a Luria-Bertani (LB) medium, 10 ug/mL of chloramphenicol (Sigma, USA) and xylos (TCI, Tokyo) for deriving expression of biosynthesis genes were added to be 1%, and culturing was performed while 37°C and 200 rpm were maintained for 72 hours. In order to confirm metabolites produced by the biosynthesis genes, Bacillus subtilis strains transformed by each plasmid were extracted using methanol, and confirmed by UPLC-MS (Ultra Performance Liquid Chromatography Mass Spectrometry). The results are shown in FIG. 4, and when the masses of the detected materials were confirmed, material no. 1 showed a value of 1142.6, material no. 2 showed a value of 1156.5, and material no. 3 showed a value of 1158.3.

### 4) Separation and purification of expressed metabolites

Materials synthesized from Bacillus transformed by Plasmids 1 and 2 were separated and purified, respectively. After expressing a synthesis gene by xylos, 2 L of a cell culture medium was centrifuged at 4,000 xg for 20 minutes to obtain cells, a supernatant was discarded, resuspension was performed using 100 mL of methanol, and it was allowed to stand at room temperature for 20 minutes. Thereafter, centrifugation was performed at 12,000 xg for 15 minutes to secure an extract.

The secured methanol extract was dehydrated with anhydrous sodium sulfate and concentrated under reduced pressure. It was dissolved in methanol/dichloromethane (1/9 (v/v)) again, an organic layer was filtered, and preparative column chromatography (Prep-HPLC) (5%-70% acetonitrile (0.1% TFA): 95%-30% water (0.1% TFA) ) was used to obtain a compound. In order to prove the structures of the obtained two materials, a 600Mhz nuclear magnetic resonator was used to obtain a ¹H NMR spectrum as follows.

Material No. 1 shown in FIG. 4 was obtained as a white solid compound, and its ¹H NMR was confirmed to be identical to that of a natural product Micrococcin P2.

<Material No. 1> ¹H NMR (600 MHz, MeOD) δ 8.40 - 8.31 (m, 4H), 8.26 (s, 1H), 8.21 (s, 1H), 8.14 (s, 1H), 7.95 (s, 1H), 6.83 (q, *J* = 7.1 Hz, 1H), 6.60 (q, *J* = 6.8 Hz, 1H), 5.25 (d, *J* = 2.9 Hz, 1H), 5.21 (d, *J* = 8.9 Hz, 1H), 4.77 (d, *J* = 3.1 Hz, 1H), 4.63 - 4.56 (m, 1H), 4.30 (qd, *J* = 6.3, 2.8 Hz, 1H), 4.10 (d, *J* = 13.0 Hz, 1H), 2.63 (dq, *J* = 13.4, 6.7 Hz, 1H), 2.19 (s, 3H), 2.20 - 2.14 (m, 1H), 1.93 (s, 1H) 1.85 (app. d, *J* = 7.0 Hz, 6H), 1.55 (d, *J* = 6.4 Hz, 3H), 1.18 (d, *J* = 6.6 Hz, 3H), 1.13 (d, *J* = 6.6 Hz, 3H), 0.99 (d, *J* = 6.9 Hz, 3H); LCMS: 1142.2 (M+H⁺) for C₄₈H₄₈N₁₃O₉S₆.

Material No. 2 shown in FIG. 4 was obtained as a yellow solid compound, and its ¹H NMR was confirmed to be identical to that of a natural product Thiocillin IV.

<Material No. 2> ¹H NMR (600 MHz, CDCl₃) δ 8.71 (s, 1H), 8.57(s, 1H), 8.30 (d, *J* = 8.0 Hz, 1H), 8.26 (s, 1H), 8.20 (s, 1H), 8.17 - 8.14 (m, 2H), 8.08 - 7.99 (m, 3H), 7.92 (s, 1H), 7.79 (d, *J* = 7.1 Hz, 1H), 7.74 (s, 1H), 6.89 (d, *J* = 7.0 Hz, 1H), 6.49 (q, *J* = 6.9 Hz, 1H), 5.40 (d, *J* = 10.2 Hz, 1H), 5.25 (t, *J* = 9.7 Hz, 1H), 4.96 (dd, *J* = 6.8, 3.5 Hz, 1H), 4.49 (s, 1H), 4.46 (s, 1H), 4.15(m, 1H), 2.85(s, 3H), 2.53 (ddd, *J* = 16.3, 13.4, 6.7 Hz, 1H), 2.28 (s, 3H), 1.86 (d, *J* = 7.0 Hz, 6H), 1.69 (d, *J* = 6.3 Hz, 3H), 1.20 (d, *J* = 6.6 Hz, 3H), 1.16 (d, *J* = 6.3 Hz, 3H), 1.00 (d, *J* = 6.6 Hz, 3H) ; LCMS: 1156.6 for C₄₉H₄₉N₁₃O₉S₆.

Meanwhile, Material No. 3 shown in FIG. 4 showed a mass value of 1158.3 as detected by UPLC-MS, and was confirmed to have a mass identical to that of YM-266183.

The structures of the materials biosynthesized by each plasmid are shown in the following Table 4.

**[Table 4] Structures of materials biosynthesized from transformed Bacillus**

| Plasmid | Plasmid 1 | Plasmid 2 | Plasmid 3 |
|---|---|---|---|
| Structures of biosynthesize d materials | 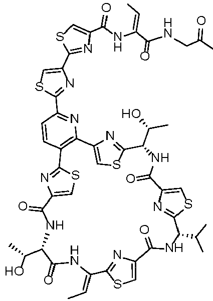 | 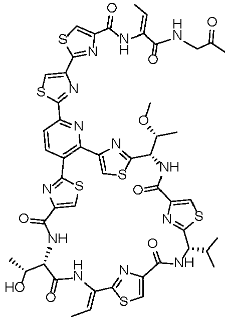 | 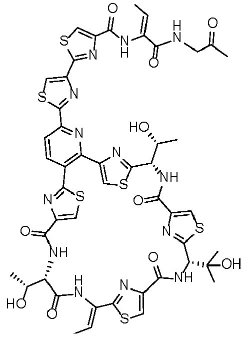 |

Each compound in Table 4 showed the structures of Micrococcin P2, Thiocillin IV, and YM-266183 synthesized by Plasmids 1, 2, and 3, in order.

Into Plasmid 1, biosynthesis genes BcTclE, I, J, K, L, M, N, and P were inserted, and a natural product Micrococcin P2 was synthesized therefrom. Plasmid 1 was a gene cluster which may biosynthesize Micrococcin P2, and it was shown that the skeleton of the natural product Micrococcin P2 was able to be synthesized by biosynthesis genes forming Plasmid 1.

Plasmid 2 was Plasmid 1 into which a BcTclO gene was further inserted, and Thiocillin IV was obtained therefrom. That is, it was shown that when a Micrococcin P2 biosynthesis gene and a BcTclO gene were inserted together, selectively O-methylated Thiocillin IV was synthesized.

Plasmid 3 was Plasmid 1 into which BcTclD and BcTclC genes were further inserted, thereby obtaining a Valine hydroxylated material, YM-266183. That is, it was shown that Bacillus which expresses a Micrococcin P2 gene and BcTclD and C genes together was cultured, separated, and purified, thereby obtaining a YM-266183 compound.

The biosynthesis genes may adjust modification by biosynthesis enzymes inside a microcycle through a combination between TclO or TclD,C and a natural product Micrococcin P2, as confirmed in each plasmid example.

### [Example 3] Preparation of micrococcin-based carboxylic acid compound C-1

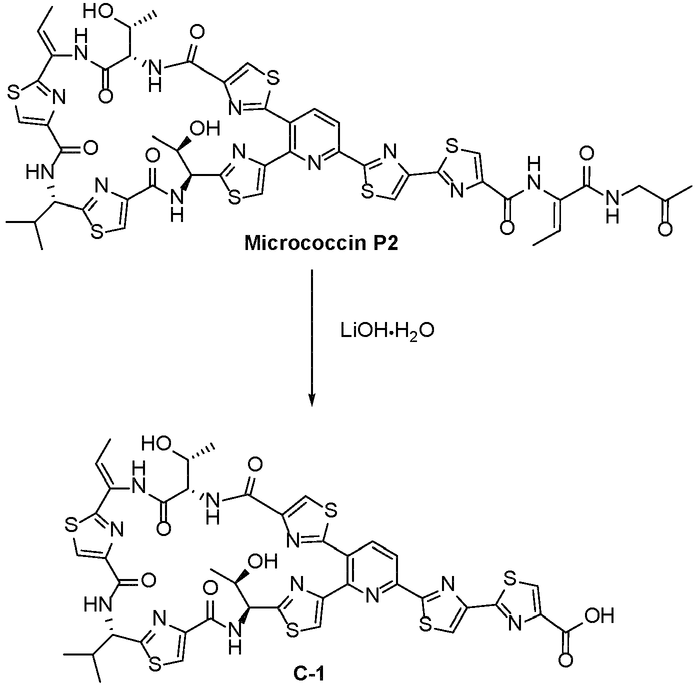

Micrococcin P2 (14 mg, 0.012 mmol) was added to a mixed solvent of tetrahydrofuran (150 µL)/ water (150 pL), lithium hydroxide monohydrate (LiOH·H₂O) (4.0 mg, 0.096 mmol) was added thereto, and stirring was performed at 50°C for 17 hours. After completing the stirring, the reaction mixture was diluted with dichloromethane, washed with a 1 M hydrochloric acid solution, and extracted with a super solvent (chloroform: propanol = 4:1 (v:v)). Thereafter, dehydration was performed using anhydrous sodium sulfate, preparative column chromatography (Prep-HPLC) (5%-70% acetonitrile (0.1% TFA): 95%-30% water (0.1% TFA)) was used to perform separation, thereby obtaining a light yellow solid compound C-1 (7.4 mg, 61%).

¹H NMR (400 MHz, DMSO, ppm) δ 9.55 (s, 1H), 8.59 - 8.51 (m, 2H), 8.50 - 8.35 (m, 3H), 8.35 - 8.27 (m, 2H), 8.28 - 8.17 (m, 2H), 8.13 (s, 1H), 7.88 (d, J = 7.6 Hz, 1H), 6.47 (q, J = 6.5 Hz, 1H), 5.20 - 4.96 (m, 2H), 4.73 - 4.63 (m, 1H), 4.42 - 4.33 (m, 1H), 4.04 - 3.96 (m, 1H), 1.76 (d, J = 6.3 Hz, 3H), 1.38 (d, J = 6.0 Hz, 3H), 1.00 (dd, J = 25.3, 6.1 Hz, 6H), 0.86 (d, J = 6.4 Hz, 3H); LCMS: 1004.2 (M+H⁺) for C₄₁H₃₇N₁₁O₈S₆.

### [Example 4] Preparation of micrococcin-based carboxylic acid compound C-2

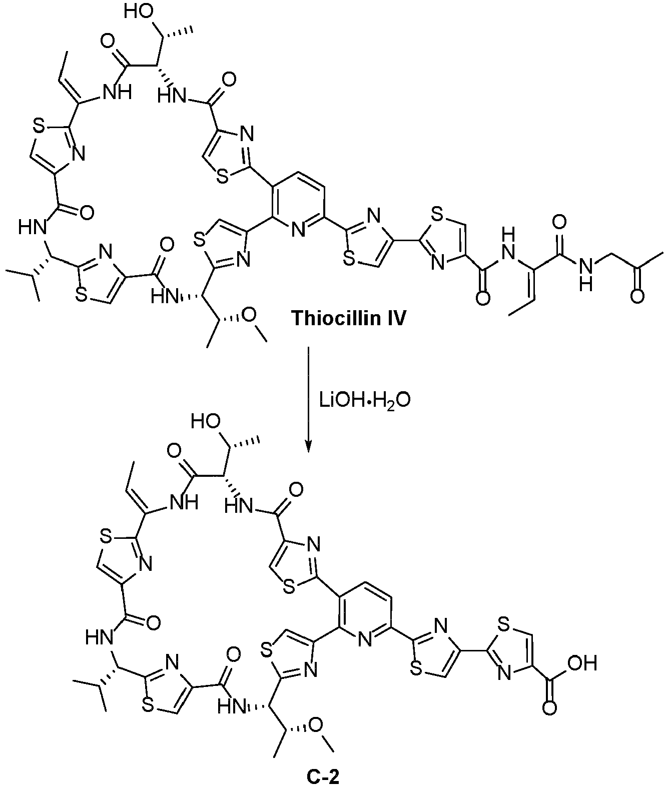

A carboxylic acid compound C-2 was prepared by the same reaction as in Example 3, except that Thiocillin IV was used instead of Micrococcin P2 of Example 3, which was confirmed by LCMS. LCMS: C₄₂H₄₀N₁₁O₈S₆ 1018.2 (M+H⁺)

### [Example 5] Preparation of Micrococcin derivative 9

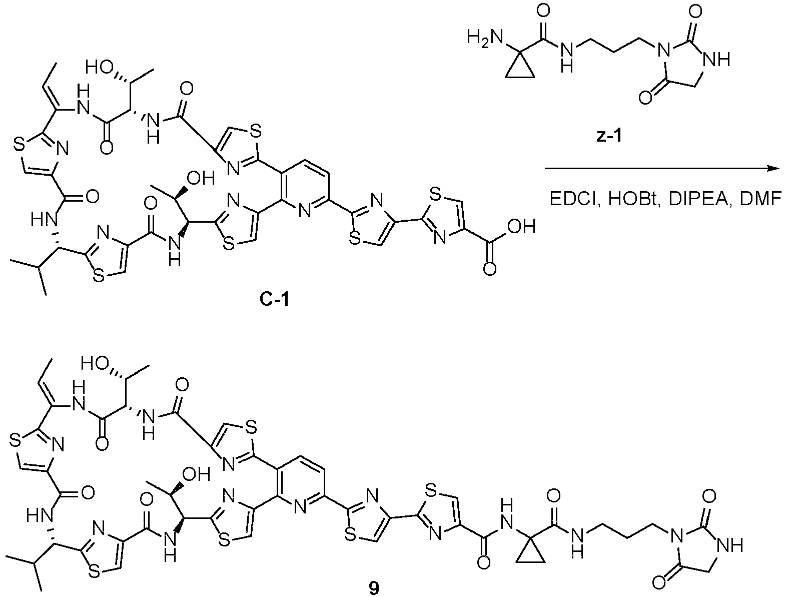

The micrococcin-based carboxylic acid compound C-1 (30 mg, 0.03 mmol) prepared in Example 3 was dissolved in dimethyl formamide (0.3 mL), 1-ethyl-3-(3-dimethylaminopropyl)carbodimide (7.5 mg, 0.039 mmol, 1.3 eq), hydroxybenzotriazole (5.3 mg, 0.039 mmol, 1.3 eq), N,N-diisopropylethylamine (21 uL, 0.12 mmol, 4 eq), and amine compound z-1 (8.6 mg, 0.036 mmol, 1.2 eq) were added, and stirring was performed at room temperature for 12 hours. After completing stirring, the solution was diluted with dichloromethane, an organic layer was washed with water three time, and was dehydrated with anhydrous sodium sulfate to be concentrated under reduced pressure. The obtained residue was separated using column chromatography with a (3-5 % methanol: 97-95% dichloromethane) solution to obtain Micrococcin derivative 9 (15.1 mg, 41%) as a white solid.

¹H NMR (400 MHz, CDCl₃) δ 8.58 (s, 1H), 8.39 (d, *J* = 9.6 Hz, 1H), 8.29 (d, *J* = 8.1 Hz, 1H), 8.26 - 8.15 (m, 4H), 8.14 - 8.09 (m, 2H), 8.01 - 7.94 (m, 4H), 7.40 (t, *J* = 6.2 Hz, 1H), 6.42 (q, *J* = 7.0 Hz, 1H), 5.88 (s, 1H), 5.29 - 5.18 (m, 2H), 4.87 (d, *J* = 7.9 Hz, 1H), 4.64 (s, 1H), 4.50 (s, 1H), 4.40 (d, *J* = 6.6 Hz, 1H), 3.93 (s, 2H), 3.63 (t, *J* = 6.1 Hz, 2H), 3.32 - 3.22 (m, 2H), 2.96 (s, 1H), 2.54 - 2.42 (m, 1H), 1.92 - 1.69 (m, 7H), 1.57 (d, *J* = 6.3 Hz, 3H), 1.23 - 1.16 (m, 5H), 1.15 (d, *J* = 6.7 Hz, 3H), 0.97 (d, *J* = 6.6 Hz, 3H) ; LCMS: 1226.23 (M+H⁺) for C₅₁H₅₁N₁₅O₁₀S₆.

### Sequence listing free text

The sequence listing is attached as a separate file.

## Claims

1. A method for preparing a micrococcin-based carboxylic acid compound represented by the following Chemical Formula 1 by reacting a micrococcin compound represented by the following Chemical Formula 2 with a metal hydroxide: 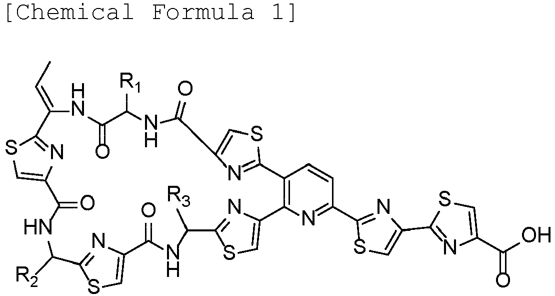 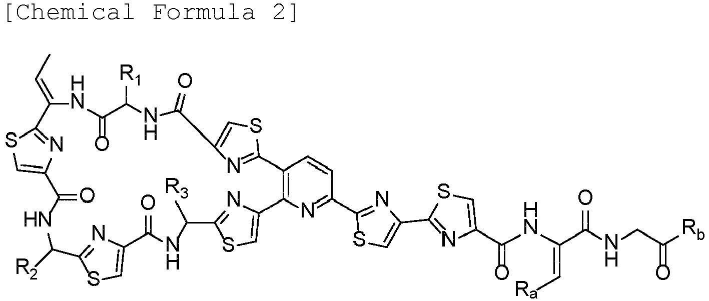 wherein
R₁ to R₃ are independently of one another hydrogen, C1-C10 alkyl, C3-C10 cycloalkyl, C6-C12 aryl, or C3-C10 heteroaryl;
the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl;
Rₐ is hydrogen, C1-C10 alkyl, C2-C10 alkenyl, or C1-C10 alkoxyC1-C10 alkyl; and
R_{b} is hydrogen or C1-C10 alkyl.

2. The method for preparing a micrococcin-based carboxylic acid compound of claim 1, wherein R₁ to R₃ are independently of one another hydrogen or C1-C10 alkyl, and the alkyl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl.

3. The method for preparing a micrococcin-based carboxylic acid compound of claim 1, wherein Rₐ is hydrogen or C1-C10 alkyl; and R_{b} is C1-C10 alkyl.

4. The method for preparing a micrococcin-based carboxylic acid compound of claim 2, wherein R₁ to R₃ are independently of one another hydrogen or *-L₁-R₄, in which L₁ is C1-C3 alkylene, and R₄ is hydrogen, hydroxy, thio, C1-C4 alkylthio, C1-C4 alkyl, C1-C4 alkoxy, C6-C12 aryl, hydroxyC6-C12 aryl, or C3-C10 heteroaryl.

5. The method for preparing a micrococcin-based carboxylic acid compound of claim 4, wherein R₁ to R₃ are independently of one another hydrogen, or selected from the following structures:
*-CH₃ *-CH₂OH *-CHCH₃OH *-CHCH₃OCH₃ *-CH₂SH *-C(CH₃)₂OH
*-C(CH₃)₃ *-CH(CH₃)₂ *-CH_{2CH}(_{CH3})₂ *-CHCH₃CH₂CH₃ *-CH₂CH₂SCH₃

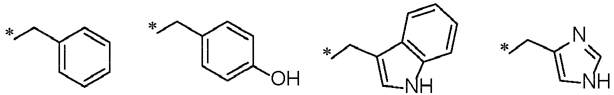

6. The method for preparing a micrococcin-based carboxylic acid compound of claim 1, wherein the micrococcin compound of Chemical Formula 2 is represented by the following Chemical Formula 2-1-A, 2-1-B, or 2-1-C:

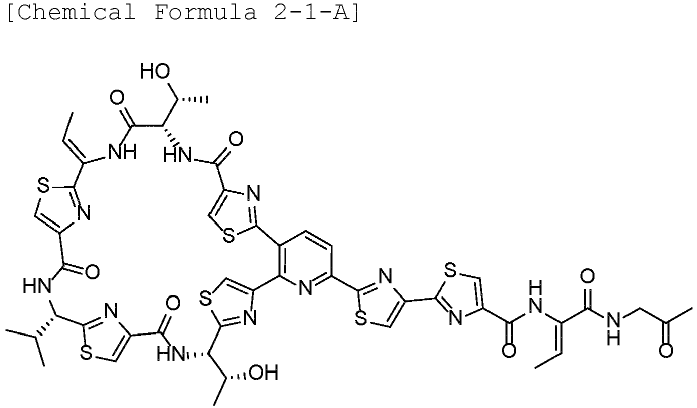

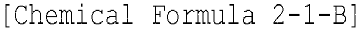

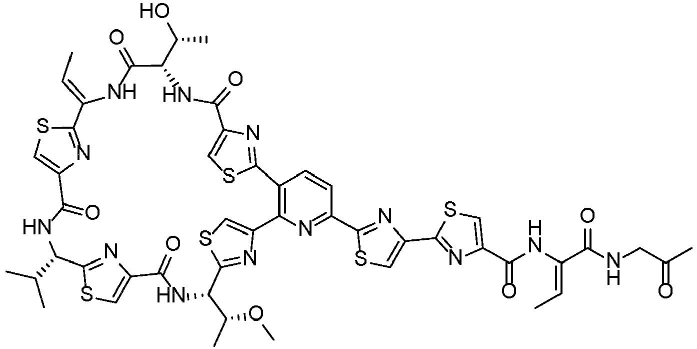

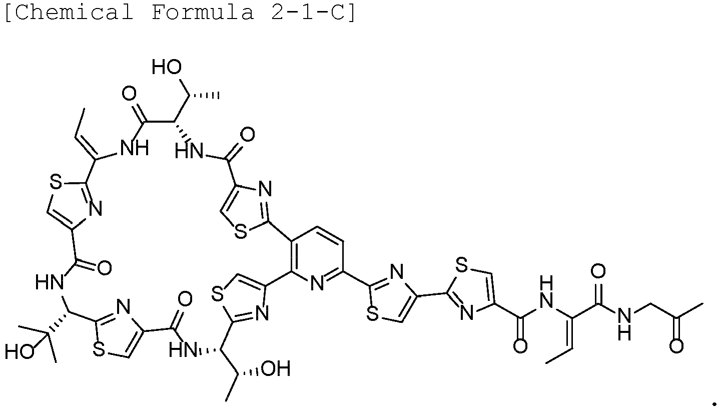

7. The method for preparing a micrococcin-based carboxylic acid compound of claim 1, wherein a metal of the metal hydroxide is an alkaline metal or an alkali earth metal.

8. The method for preparing a micrococcin-based carboxylic acid compound of claim 1, wherein the metal hydroxide is used at 3 to 12 mol with respect to 1 mol of the micrococcin compound of Chemical Formula 2.

9. The method for preparing a micrococcin-based carboxylic acid compound of claim 1, wherein the micrococcin compound of Chemical Formula 2 is separated from a natural product or prepared by synthesis.

10. The method for preparing a micrococcin-based carboxylic acid compound of claim 6, wherein the micrococcin compound of Chemical Formula 2-1-A is prepared by:
1) producing an expression vector including BcTclE, BcTclI, BcTclJ, BcTclK, BcTclL, BcTclM, BcTclN, and BcTclP genes; and
2) transforming Bacillus subtilis with the expression vector of 1).

11. The method for preparing a micrococcin-based carboxylic acid compound of claim 6, wherein the micrococcin compound of Chemical Formula 2-1-B is prepared by:
1) producing an expression vector including BcTclE, BcTclO, BcTclI, BcTclJ, BcTclK, BcTclL, BcTclM, BcTclN, and BcTclP genes; and
2) transforming Bacillus subtilis with the expression vector of 1).

12. The method for preparing a micrococcin-based carboxylic acid compound of claim 6, wherein the micrococcin compound of Chemical Formula 2-1-C is prepared by:
1) producing an expression vector including BcTclE, BcTclD, BcTclC, BcTclI, BcTclJ, BcTclK, BcTclL, BcTclM, BcTclN, and BcTclP genes; and
2) transforming Bacillus subtilis with the expression vector of 1).

13. A method for preparing a micrococcin compound of the following Chemical Formula 2-2, the method comprising:
1) producing an expression vector including BcTclE, BcTclI, BcTclJ, BcTclK, BcTclL, BcTclM, BcTclN, and BcTclP genes; and
2) transforming Bacillus subtilis with the expression vector of 1): 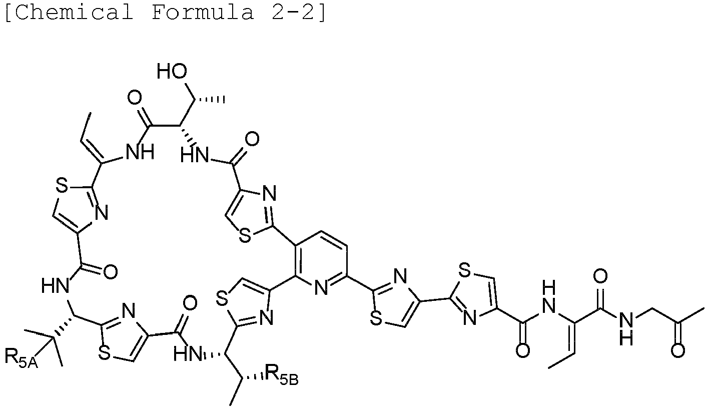 wherein
R_{5A} is hydrogen or hydroxy, and R_{5B} is hydroxy or methoxy.

14. The method for preparing a micrococcin compound of claim 13, wherein the expression vector further includes one or more of BcTclO, BcTclD, and BcTclC genes.

15. A micrococcin-based carboxylic acid compound represented by the following Chemical Formula 1: 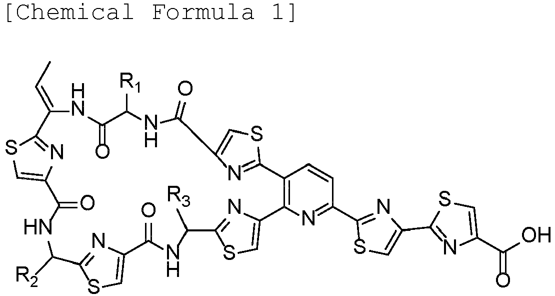 wherein
R₁ to R₃ are independently of one another hydrogen, C1-C10 alkyl, C3-C10 cycloalkyl, C6-C12 aryl, or C3-C10 heteroaryl; and
the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl.

16. A method for preparing a micrococcin derivative, the method comprising:
reacting a micrococcin compound of the following Chemical Formula 2 with a metal hydroxide to prepare a micrococcin-based carboxylic acid compound of the following Chemical Formula 1; and
preparing a micrococcin derivative of the following Chemical Formula 3 from the micrococcin-based carboxylic acid compound of the following Chemical Formula 1: 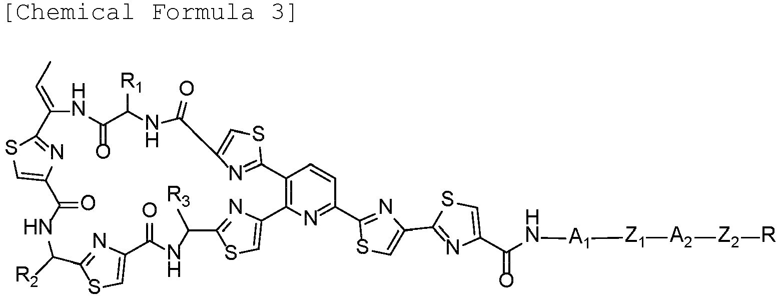 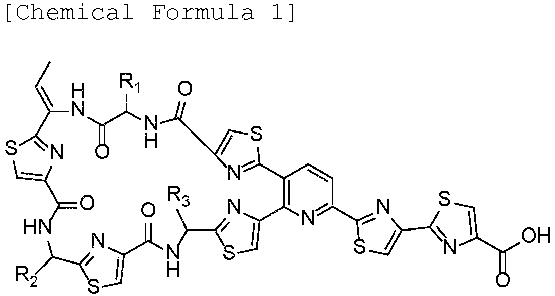 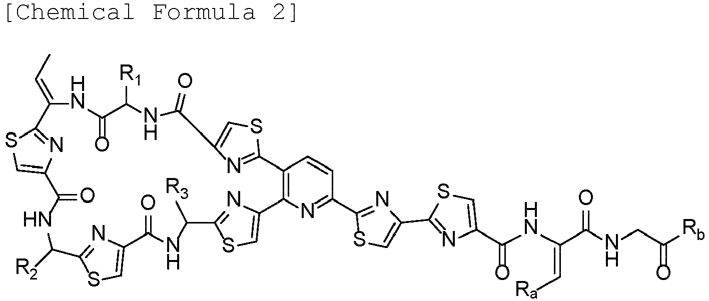 wherein
R₁ to R₃ are independently of one another hydrogen, C1-C10 alkyl, C3-C10 cycloalkyl, C6-C12 aryl, or C3-C10 heteroaryl; and
the alkyl, cycloalkyl, aryl, and heteroaryl of R₁ to R₃ may be further substituted by one or more selected from the group consisting of hydroxy, thio, C1-C10 alkylthio, C1-C10 alkyl, C1-C10 alkoxy, hydroxyC1-C10 alkyl, C6-C12 aryl, hydroxyC6-C12 aryl, and C3-C10 heteroaryl;
Rₐ is hydrogen, C1-C10 alkyl, C2-C10 alkenyl, or C1-C10 alkoxyC1-C10 alkyl; and
R_{b} is hydrogen or C1-C10 alkyl;
Z₁ and Z₂ are independently of each other a single bond, -CONR₁₁-, -NR₁₂CO-, -COO-, -OCO-, -CR₁₃R₁₄-, -NR₁₅COO-, -NR₁₆-, -S-, -O-, -SO₂-, or -OCONR₁₇-, in which R₁₂ to R₁₇ are independently of one another hydrogen, hydroxy, C1-C10 alkyl, carboxylC1-C10 alkyl, or C1-C10 alkoxycarbonylC1-C10 alkyl;
A₁ is 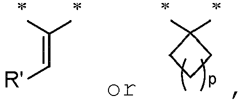 in which R' is hydrogen, C1-C10 alkyl, C2-C10 alkenyl, or C1-C10 alkoxyC1-C10 alkyl, and p is an integer of 0 to 4;
A₂ is a single bond, C1-C10 alkylene, C3-C10 cycloalkylene, C3-C10 heterocycloalkylene, C6-C20 arylene, or C6-C20 heteroarylene; and
R is hydrogen, halogen, amino, hydroxy, -B(OH)₂, substituted or unsubstituted haloC1-C10 alkyl, substituted or unsubstituted C1-C10 alkyl, substituted or unsubstituted C2-C10 alkenyl, substituted or unsubstituted C3-C10 cycloalkyl, substituted or unsubstituted C3-C10 heterocycloalkyl, substituted or unsubstituted C6-C20 aryl, or substituted or unsubstituted C3-C20 heteroaryl.

17. The method for preparing a micrococcin derivative of claim 16, wherein in Chemical Formula 3, Z₁ is a single bond, -CONR₁₁-, -NR₁₂CO-, or -COO-; Z₂ is a single bond, -CONR₁₁-, - NR₁₂CO-, -NR₁₅COO-, -NR₁₆-, -S-, or -OCONR₁₇-; R₁₁, R₁₂, and R₁₅ to R₁₇ are independently of one another hydrogen, hydroxy, C1-C10 alkyl, C1-C10 alkoxycarbonylC1-C10 alkyl, or carboxylC1-C10 alkyl.

18. The method for preparing a micrococcin derivative of claim 16, wherein in Chemical Formula 3,
A₁ is 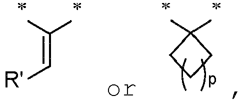 in which R' is C1-C10 alkyl, and p is an integer of 0 to 2;
A₂ is a single bond or C₁-C10 alkylene;
R is hydrogen, amino, or -B(OH)₂, or any one selected from the following structures: 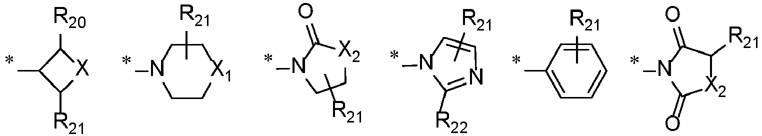 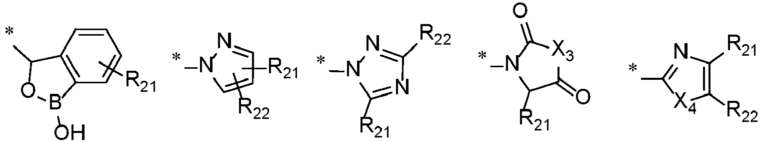 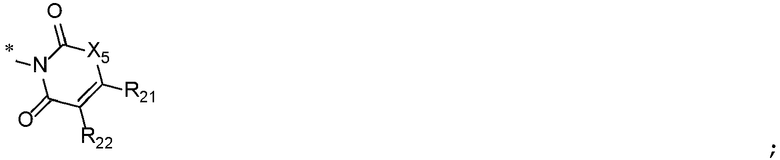
X is O or S;
X₁ is NR₃₁, O, S, or SO₂;
X₂ to X₅ are independently of one another NR₃₂, O, or S; and
R₂₀ to R₂₂, R₃₁, and R₃₂ are independently of one another hydrogen, halogen, nitro, C1-C10 alkylcarbonyl, C1-C10 alkoxycarbonyl, C1-C10 alkyl, C1-C10 alkylsulfonyl, aminosulfonyl, hydroxyC1-C10 alkyl, dihydroxyC1-C10 alkyl, cyanoC1-C10 alkyl, C3-C10 cycloalkyl, C1-C10 alkoxycarbonylC1-C10 alkyl, or carboxylC1-C10 alkyl.
